(19) Europäisches Patentamt
European Patent Office
Office européen des brevets

(11) EP 1 224 945 A1

(12) EUROPEAN PATENT APPLICATION
published in accordance with Art. 158(3) EPC

(43) Date of publication:
**24.07.2002 Bulletin 2002/30**

(21) Application number: **00969999.2**

(22) Date of filing: **23.10.2000**

(51) Int Cl.[7]: **A61K 45/00**, A61K 31/4166, A61K 31/513, A61P 27/10, A61P 43/00

(86) International application number:
**PCT/JP00/07390**

(87) International publication number:
**WO 01/30388 (03.05.2001 Gazette 2001/18)**

(84) Designated Contracting States:
**AT BE CH CY DE DK ES FI FR GB GR IE IT LI LU MC NL PT SE**
Designated Extension States:
**AL LT LV MK RO SI**

(30) Priority: **25.10.1999 JP 30307699**

(71) Applicant: **Senju Pharmaceutical Co., Ltd.**
**Osaka-shi, Osaka 541-0046 (JP)**

(72) Inventors:
- **TSUZUKI, Masakatsu**
  **Kyoto-shi, Kyoto 606-8107 (JP)**
- **WAKI, Mitsunori**
  **Kobe-shi, Hyogo 651-2111 (JP)**
- **KAWAMOTO, Yoko**
  **Kobe-shi, Hyogo 651-2116 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

# (54) TENSION-RELIEVING AGENTS FOR CILIARY MUSCLE

(57) Use of a chymase inhibitor in preventing and treating nearsightedness caused by the continuous tension of ciliary muscles makes it possible to achieve exact preventive and therapeutic effects with little side effects.

EP 1 224 945 A1

## Description

### TECHNICAL FIELD

The present invention relates to a ciliary muscle relaxant. More particularly, the invention relates to a prophylactic or therapeutic composition for myopia inclusive of pseudomyopia arising from hypertonia of the ciliary muscle. The invention further provides novel uses for chymase inhibitors.

### BACKGROUND ART

The ciliary muscle, which is an intraocular smooth muscle, adjusts the thickness of the crystalline lens by undergoing contraction and relaxation for accommodation necessary for causing the light beam incident on the eye to focus on the retina. In view of the recent increase in the number of people with myopia owing to prolongation of study time and the high incidence of myopia among highly educated persons and workers engaged in many hours of near work, it has been suggested that environmental factors are involved in the onset of myopia and that, among such factors, excessive near work is a risk factor for myopia [Curtin B. J., The Myopias. Harper and Row. Philadelphia, 61-151 (1985); Simensen B. et al., Acta. Ophthalmol., 72, 469-471 (1994); Tay M. T. et al., Ann. Acad. Med., Singapore, 21, 785-791 (1992); Teasdale T. W. et al., The Lancet 2, 1351-1354 (1988); M. Rosner et al., Arch. Ophthalmol., 105, 1508-1511 (1987)].

In near work, contraction of the ciliary muscle increases the thickness of the lens or displaces the lens toward the anterior chamber to thereby allow the light incident on the eye to focus on the retina. Excessive near work leads to a chronic elevation of the tonus of the ciliary muscle and as this hypertonic state persists, the ciliary muscle atrophies to induce atrophy of the choroid and extension of the axial length of the eye. It is suspected that, as a consequence, the light incident on the eye focuses anteriorly of the retina, thus giving rise to myopia [Otsuka J., Acta. Soc., Ophthalmol. Jpn., 86, 2067-2091 (1982)].

In patients with mild myopia, the extended axial length of the eye is paralleled by disturbance of the blood-ciliary barrier and this suggests that certain functional or organic changes have already occurred in the ciliary muscle in the incipient stage of myopia [Hosaka, Akira et al., Clinical Ophthalmology, 39, 569-578 (1985)]. Moreover, chronic near work tends to bring the ciliary muscle into spastic state and induce transient positive accommodation myopia.

In view of the foregoing, it is generally considered that myopia may be cured by ameliorating the chronic contraction of the ciliary muscle. For this reason, topical administration (instillation) of a drug such as tropicamide, which is a parasympatholytic/antimuscarinic drug, has so far been tried to attain relaxation of the ciliary muscle but its efficacy varies with different patients and is not fully satisfactory. Further, as physical therapies, low-frequency-wave treatment, ultrasonic therapy, distant accommodation training, etc. are being practiced but none have proved successful. Aside from the above, as a surgical therapy, the procedure to reduce the refractive power of the cornea has also been tried but no satisfactory effect has been obtained but rather cases of lapsing into hyperopia and serious side effects have been reported.

Thus, it is the state of the art that no satisfactory method for prophylaxis or therapy of myopia nor a satisfactory prophylactic or therapeutic drug for myopia is available today. Therefore, development of a useful protocol for prophylaxis or therapy of myopia and a useful relevant prophylactic or therapeutic drug is being awaited by both the ophthalmologist and the patient.

### DISCLOSURE OF INVENTION

It is an object of the present invention to provide a ciliary muscle relaxant for the prophylaxis or therapy of myopia, which reduces the elevated tension of the ciliary muscle, a conjectured causative factor in myopia.

After an intensive investigation made for accomplishing the above object, the inventors of the present invention found surprisingly that an inhibitor of chymase reduces the tension of the ciliary muscle to thereby exhibit efficacy in the prophylaxis or therapy of myopia inclusive of pseudomyopia. The present invention has accordingly been developed. The present invention, therefore, is directed to a ciliary muscle relaxant comprising a chymase inhibitor as an active ingredient, particularly a pharmaceutical composition for reducing the tension of the ciliary muscle which comprises a chymase inhibitor and a pharmaceutically acceptable carrier or excipient, and a method of reducing the tension of the ciliary muscle and, hence, preventing or treating myopia which comprises administering an effective amount of a chymase inhibitor to a patient who requires it, particularly to the patient's eye.

The chymase inhibitors which can be used in the present invention include all substances that inhibit the enzymatic activity of chymases belonging to the serine protease series, and particularly inhibitors of human chymase activity, namely inhibitors of human heart chymase, human mast cell chymase, or human skin chymase can be used with advantage.

As substances having such chymase inhibitory activity, chymostatin and the following substances are known.

Thus, USP5314815 discloses the adduct of a serine hydrase to a phosphate or phosphonate of the formula (I)

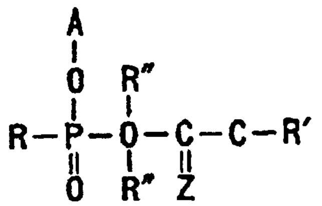

(I)

(wherein R represents alkyl or alkoxy; A represents phenyl or naphtyl; R' represents phenyl or alkyl; R" represents hydrogen or methyl; Z represents an electronegative group such as O, N, S, OH, $NH_2$, or the like) and, as specific examples of the compound represented by said formula (I), mentions 4-nitrophenyl phenacyl methylphosphate, 4-nitrophenyl-4-nitrophenacyl methylphosphate, 4-nitrophenyl-4-methylphenacyl methylphosphate, 4-nitrophenyl-4-methoxyphenacyl methylphosphate and 4-nitrophenyl-4-chlorophenacyl methylphosphate.

USP 5306824 discloses a compound represented by the formula (II)

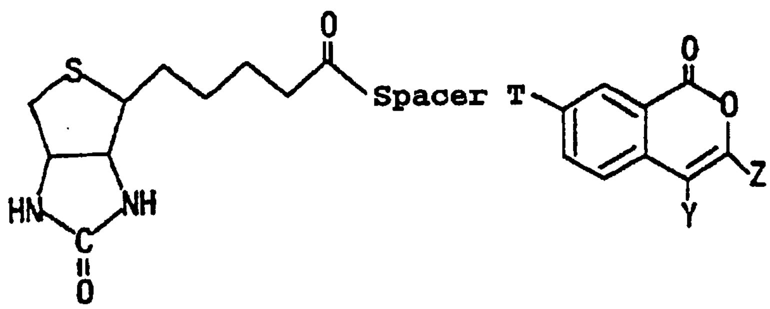

(II)

(wherein Z is selected from the group consisting of H, halogen, $C_{1-6}$ alkyl, $C_{1-6}$ fluorinated alkyl, $C_{1-6}$ alkyl substituted with $R^1$, $C_{1-6}$ fluorinated alkyl substituted with $R^1$, $C_{1-6}$ alkoxy, $C_{1-6}$ fluorinated alkoxy, $C_{1-6}$ alkoxy substituted with $R^1$, fluorinated alkoxy substituted with $R^1$, $C_{1-6}$ alkyl having a phenyl group, $C_{1-6}$ alkoxy having a phenyl group, $C_{1-6}$ alkyl having a phenyl group substituted with $R^2$, $C_{1-6}$ alkyl having a phenyl group substituted with two $R^2$ groups, $C_{1-6}$ alkoxy having a phenyl group substituted with $R^2$, and $C_{1-6}$ alkoxy having a phenyl group substituted with two $R^2$ groups; $R^2$ represents halogen, COOH, OH, CN, $NO_2$, $NH_2$, $C_{1-6}$ alkyl, $C_{1-6}$ alkoxy, $C_{1-6}$ alkylamine, $C_{1-6}$ dialkylamine, $C_{1-6}$ alkyl-O-CO-, $C_{1-6}$ alkyl-O-CO-NH-, or $C_{1-6}$ alkyl-S-: $R^1$ represents halogen, COOH, OH, CN, $NO_2$, $NH_2$, $C_{1-6}$ alkoxy, $C_{1-6}$ alkylamine, $C_{1-6}$ dialkylamine, $C_{1-6}$ alkyl-O-CO-, $C_{1-6}$ alkyl-O-CO-NH-, $C_{1-6}$ alkyl-S-, or tosylamino; Spacer is an organic structure having a length of 3-24 Ångstrome units and containing at least one bond selected from the group consisting of $-CH_2-CH_2-$, -CO-NH-, -NH-CO-, $-CH_2-CO-$, $-CH_2-NH-$, $NH-CH_2-$, and $-C_6H_4-$; T represents -NH-, -O-, or -S-; Y is selected from the group consisting of H, halogen, trifluoromethyl, methyl, OH, and methoxy). As specific examples thereof, the following are disclosed:

7-biotinylamino-4-chloro-3-(2-phenylethoxy)isocoumarin and 7-(6-biotinylaminocaproyl)amino-4-chloro-3-(2-phenylethoxy) isocoumarin.

WO 93/25574 discloses a compound of the formula (III):

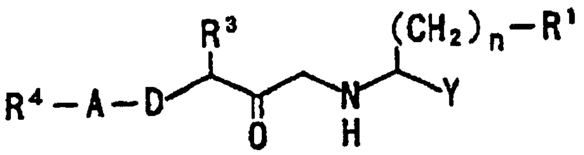

(III)

[wherein n is equal to 1 or 2;

$R^1$ represents phenyl, naphthyl, $(C_{3-7})$cycloalkyl, unsaturated heterocycle, or benzo-fused unsaturated heterocycle; where said unsaturated heterocycle is selected from among pyrrolyl, pyrrolinyl, furyl, dihydrofuryl, thienyl, dihydrothienyl, oxazolyl, oxazolidinyl, isoxazolyl, isoxazolinyl, imidazolyl, imidazolinyl, thiazolyl, thiazolidinyl, isothiazolyl, isothiazolinyl, pyrazolyl, pyrazolinyl, triazolyl, tetrazolyl, 1,3,5-oxadiazolyl, 1,2,4-oxadiazolyl, 1,3,5-thiadiazolyl, 1,2,4-thiadiazolyl, pyridyl, pyranyl, pyrazinyl, pyrimidinyl, pyridazinyl, 1,2,4-triazinyl, 1,2,3-triazinyl, 1,3,5-triazinyl, 1,2,5-thiadiazinyl, 1,2,5-oxathiazinyl, and 1,2,6-oxathiazinyl; wherein said benzo-fused unsaturated heterocycle is selected from among benzoxazolyl, benzothiazolyl, benzimidazolyl, thianaphthenyl, isothianaphthenyl, benzofuranyl, isobenzofuranyl, chromenyl, isoindolyl, indolyl, indazolyl, isoquinolyl, quinolyl, phthalazinyl, quinoxalinyl, quinazolinyl, cinnolinyl and benzoxazinyl; wherein each of said phenyl, naphthyl, unsaturated heterocycle and benzo-fused unsaturated heterocycle may optionally be substituted with one to three substituents, said substituents being independently selected from among bromo, chloro, fluoro, $(C_{1-5})$ alkyl, $(C_{1-5})$alkoxy, $(C_{1-5})$ alkylthio, $(C_{1-5})$alkylamino, $(C_{1-5})$alkylsulfonyl, $(C_{1-5})$dialkylamino, hydroxyl, amino, nitro, cyano, trifluoromethyl, $-COO(C_{1-5})$alkyl, $-CONH_2$, $-CONH(C_{1-5})$alkyl, -CON-di$(C_{1-5})$alkyl, and formyl; and wherein said $(C_{3-7})$cycloalkyl may optionally be substituted with one to three substituents, said substituents being independently selected from among bromo, chloro, fluoro, $(C_{1-5})$alkyl, $(C_{1-5})$alkylthio, $(C_{1-5})$alkoxy, hydroxyl, trifluoromethyl and oxo (O=); $R^3$ is $(C_{1-5})$alkyl, $(C_{3-6})$cycloalkyl, $(C_{3-6})$ cycloalkyl$(C_{1-5})$alkyl, $(C_{1-5})$alkoxy$(C_{1-2})$alkyl, $(C_{1-5})$alkylthio $(C_{1-2})$alkyl, phenyl, unsaturated heterocycle, phenyl$(C_{1-2})$alkyl, or unsaturated heterocycle $(C_{1-2})$alkyl; wherein said unsaturated heterocycle is as defined for $R^1$; wherein said unsaturated heterocycle $(C_{1-2})$alkyl is an unsaturated heterocycle moiety as defined in $R^1$, wherein any one of the carbon atoms of said unsaturated heterocycle moiety is substituted with $(C_{1-2})$alkyl; wherein said $(C_{1-5})$alkyl, $(C_{3-6})$cycloalkyl$(C_{1-5})$alkyl and $(C_{3-6})$ cycloalkyl may optionally be substituted with one or more fluorine atoms; wherein each of said phenyl, unsaturated heterocycle, phenyl$(C_{1-2})$alkyl, and unsaturated heterocycle$(C_{1-2})$alkyl may optionally be nuclearly substituted with one to three substituents, said substituents being independently selected from among the functionalities set forth in the definition of $R^1$ for the substituents on said phenyl;

$R^4$ is selected from among the functionalities listed in groups (a)-(d) below:

a) piperazino, piperidino, pyrrolidino, 3-azabicyclo[3.1.0]hex-3-yl, and azetidino, wherein any of the carbon atoms of said piperazino may optionally be substituted with one or two substituents, said substituents being independently selected from among $(C_{1-5})$alkyl, $(C_{1-5})$alkoxy$(C_{1-3})$alkyl, hydroxy$(C_{1-3})$alkyl, $(C_{1-5})$ alkylthio $(C_{1-3})$alkyl, amino$(C_{1-3})$alkyl, $(C_{1-5})$alkylamino$(C_{1-3})$alkyl, and $(C_{1-5})$dialkylamino$(C_{1-3})$alkyl; wherein the nitrogen in the 4-position of said piperazino may optionally be substituted with $(C_{1-5})$ alkyl, $(C_{1-5})$alkoxy$(C_{2-4})$alkyl, hydroxyl$(C_{2-4})$alkyl, amino$(C_{2-4})$alkyl, $(C_{1-5})$alkylamino$(C_{2-4})$alkyl, $(C_{1-5})$dialkylamino$(C_{2-4})$alkyl, and 2,2,2-trifluoroethyl; wherein any of the carbon atoms of said piperidino, pyrrolidino, 3-azabicyclo[3.1.0]hex-3-yl and azetidino may optionally be substituted with one or two substituents, said substituents being independently selected from among chloro, bromo, fluoro, hydroxyl, $(C_{1-5})$alkyl, amino$(C_{1-3})$alkyl, $(C_{1-5})$alkylamino$(C_{1-3})$alkyl, $(C_{1-5})$ dialkylamino$(C_{1-3})$alkyl, $(C_{1-5})$alkoxy$(C_{1-3})$alkyl, $(C_{1-5})$alkoxy, $(C_{1-5})$ alkoxy$(C_{1-3})$alkoxy, amino, $(C_{1-5})$ alkylamino, $(C_{1-5})$dialkylamino, $(C_{1-5})$alkylthio, oxo (O=), unsaturated heterocycle, azetidino, pyrrolidino, piperidino, morpholino, 4-oxopiperidino, 4-hydroxypiperidino, and piperazino, wherein the nitrogen in the 4-position of said piperazino may optionally be substituted with $(C_{1-5})$alkyl, $(C_{1-5})$alkoxy$(C_{2-4})$alkyl, hydroxy$(C_{2-4})$ alkyl, amino$(C_{2-4})$alkyl, $(C_{1-5})$alkylamino$(C_{2-4})$alkyl, $(C_{1-5})$dialkylamino$(C_{2-4})$alkyl, or 2,2,2-trifluoroethyl; wherein said unsaturated heterocycle is as defined in $R^1$; wherein said unsaturated heterocycle may optionally be substituted with one to three substituents independently selected from among the functionalities set forth in the definition of $R^1$ for the substituents on said unsaturated heterocycle;

b) 4-morpholino, 4-thiomorpholino, 1-oxothiomorpholino, or 1,1-dioxothiomorpholino; wherein any of the carbon atoms of said 4-morpholino, 4-thiomorpholino, 1-oxothiomorpholino, and 1,1-dioxothiomorpholino may optionally be substituted with one or two substituents, said substituents being independently selected from among $(C_{1-5})$alkyl, $(C_{1-5})$alkoxy$(C_{1-3})$alkyl, hydroxy$(C_{1-3})$alkyl, $(C_{1-5})$alkylthio$(C_{1-3})$alkyl, amino$(C_{1-3})$alkyl, $(C_{1-5})$alkylamino$(C_{1-3})$alkyl, and $(C_{1-5})$dialkylamino$(C_{1-3})$alkyl;

c) $(C_{1-7})$alkyl or $(C_{3-7})$cycloalkyl; wherein said $(C_{3-7})$cycloalkyl may optionally be substituted with one to three substituents, said substituents being independently selected from among halogen, hydroxyl, $(C_{1-5})$alkoxy, $(C_{1-5})$alkoxy$(C_{1-3})$alkyl, hydroxyl$(C_{1-3})$alkyl, $(C_{1-5})$alkylthio$(C_{1-3})$alkyl, amino$(C_{1-3})$alkyl, $(C_{1-5})$alkylamino $(C_{1-3})$alkyl, $(C_{1-5})$dialkylamino$(C_{1-3})$alkyl, $(C_{1-5})$alkoxy$(C_{1-3})$alkyloxy, amino, $(C_{1-5})$alkylamino, $(C_{1-5})$dialkylamino, $(C_{1-5})$alkylthio, azetidino, pyrrolidino, piperidino, piperazino, 4-$(C_{1-5})$alkylpiperazino, morpholino, thiomorpholino, oxothiomorphilino, dioxothiomorpholino, 4-oxopiperidino, 4-hydroxypiperidino, and unsaturated heterocycle, wherein said unsaturated heterocycle is as defined in $R^1$; wherein said unsaturated heterocycle may optionally be substituted with one to three substituents independently selected from among the functionalities set forth in the definition of $R^1$ for the substituents on said unsaturated heterocycle; wherein said $(C_{1-7})$ alkyl may optionally be substituted with one to three substituents, said substituents being independently selected from among halogen, hydroxyl, $(C_{1-5})$alkoxy, $(C_{1-5})$alkoxy$(C_{1-3})$alkyloxy, amino, $(C_{1-5})$alkylamino, $(C_{1-5})$

dialkylamino, $(C_{1-5})$alkylthio, azetidino, pyrrolidino, piperidino, piperazino, 4-(N)-$(C_{1-5})$alkylpiperazino, morpholino, thiomorpholino, oxothiomorpholino, dioxothiomorpholino, 4-oxopiperidino, 4-hydroxypiperidino, and unsaturated heterocycle; wherein said unsaturated heterocycle is as defined in $R^1$; wherein said unsaturated heterocycle may optionally be substituted with one to three substituents independently selected from the functionalities set forth in the definition of $R^1$ for the substituents on said unsaturated heterocycle;

d) $(R^5E)$-, wherein E is oxygen, -NH or -N$(C_{1-5})$alkyl, and wherein $R^5$ is $(C_{1-5})$alkyl, 2,2,2-trifluoroethyl, $R^7(C_{2-4})$alkyl, $R^7CO$ $(C_{2-4})$alkyl, $R^7CO$-N[$(C_{1-5})$alkyl][$(C_{2-4})$alkyl], unsaturated heterocycle$(C_{2-4})$alkyl, $(C_{1-5})$alkyl, amino$(C_{2-4})$alkyl, $(C_{1-5})$alkylamino $(C_{2-4})$alkyl, $(C_{1-5})$dialkylamino$(C_{2-4})$alkyl, $(C_{1-5})$alkoxy$(C_{2-4})$alkyl or hydroxy $(C_{2-4})$alkyl; wherein said unsaturated heterocycle$(C_{2-4})$alkyl is an unsaturated heterocycle moiety as defined in $R^1$, wherein one of the ring atoms of said unsaturated heterocycle moiety of said unsaturated heterocycle $(C_{2-4})$alkyl so defined is substituted with $(C_{2-4})$alkyl; wherein said unsaturated heterocycle$(C_{2-4})$alkyl may optionally be substituted on any of the ring atoms with one to three substituents independently selected from the functionalities set forth in the definition of $R^1$ for the substituents on said unsaturated heterocycle;

$R^7$ is azetidino, pyrrolidino, piperidino, piperazino, 4-(N)-$(C_{1-5})$alkyl piperazino, thiomorpholino, oxothiomorpholino, dioxothiomorpholino or morpholino;

A is carbonyl or sulfonyl;

D is NH, N$(C_{1-5})$alkyl, $CH_2$, oxygen, CH(OH), or CH-O- $(C_{1-5})$alkyl;

X is proline, 2-piperidinecarboxylic acid or 2-azetidinecarboxylic acid, wherein said proline, 2-piperidinecarboxylic acid and 2-azetidinecarboxylic acid may optionally be substituted with one or two substituents, said substituents being independently selected from bromine, chlorine, fluorine, $(C_{1-5})$alkyl, $(C_{1-3})$alkoxy, oxo, and hydroxy;

Y is $BF_2$, $B(OM)_2$, -CO-Z or -$C(OH)_2$Z, wherein M is hydrogen or $(C_{1-5})$alkyl, wherein the two M substituents may optionally form, together with the boron atom and the two oxygen atoms to which they are attached, a saturated heterocyclic ring containing the boron atom, 2 oxygen atoms and 2 or 3 carbon atoms, and wherein any of the carbon atoms of said heterocyclic ring may optionally be substituted with one or two $(C_{1-5})$alkyl groups;

Z is $CF_2R^{11}$, $CF_2$CO-N $R^{12}R^{13}$, -CO-N $R^{12}R^{13}$, -$COOR^{12}$ or a heterocycle selected from 2-oxazolyl, 2-thiazolyl, 2-imidazolyl, 2-thienyl, 2-furyl, 2-pyrrolyl, 5-tetrazolyl, 2-benzothiazolyl, 2-benzoxazolyl, 2-benzimidazolyl, 2-benzofuryl, 2-benzothienyl and 2-indolyl; wherein said heterocycle may optionally be substituted with one to three substituents, said substituents being independently selected from among $(C_{1-3})$alkoxy, bromo, chloro, fluoro, $(C_{1-3})$alkyl, hydroxyl, amino, nitro, cyano, -COO$(C_{1-5})$alkyl, -$CONH_2$, formyl, $(C_{1-5})$alkylthio, $(C_{1-5})$alkylamino, $(C_{1-4})$alkyl-$SO_2$-, trifluoromethyl, and $(C_{1-5})$dialkylamino;

$R^{11}$ is hydrogen, fluorine, $(C_{1-5})$alkyl, $(C_{1-6})$perfluoroalkyl, amino$(C_{1-5})$alkyl, $(C_{1-5})$alkylamino$(C_{1-5})$alkyl, di$(C_{1-5})$alkylamino $(C_{1-5})$alkyl, $(C_{1-5})$alkoxy$(C_{1-5})$alkyl, or hydroxy$(C_{1-5})$alkyl;

$R^{12}$ and $R^{13}$ are independently selected from among hydrogen, $(C_{1-5})$alkyl, $(C_{3-5})$alkenyl, and $R^7(C_{2-4})$alkyl, wherein $R^7$ is defined as above;

with the proviso that (a) no carbon alpha to a ring nitrogen in the substituent $R^4$ may be directly bound to a halogen, oxygen or nitrogen substituent, (b) when X is substituted proline, 2-piperidinecarboxylic acid or 2-azetidinecarboxylic acid, then no fluorine, oxo, $(C_{1-3})$alkoxy or hydroxyl substituent is present on either of the ring carbon atoms adjacent to the nitrogen atom of said proline, 2-piperidinecarboxylic acid or 2-azetidinecarboxylic acid, and (c) the compound of formula III cannot be defined as a compound wherein n is equal to 1, $R^1$ is phenyl, $R^3$ is phenyl $(C_{1-2})$alkyl, $R^4$ is $(R^5E)$- wherein E is oxygen and $R^5$ is $(C_{1-5})$alkyl, A is carbonyl, D is NH, X is proline and Y is $B(OM)_2$.]

As specific examples of the above compound, there are mentioned:

N-[(1,1-dimethylethoxy)carbonyl]-L-alanyl-N-[2,3-dioxo-3-methoxy-1-(phenylmethyl)propyl]-L-prolinamide;

N-[(1,1-dimethylethoxy)carbonyl]-L-valyl-N-[2,3-dioxo-3-methoxy-1-(phenylmethyl)propyl]-L-prolinamide;

N-[4-[N-methylamino]piperidine-1-carbonyl]-L-valyl-N-[3,3,3-trifluoro-2-oxo-1(S)-(phenylmethyl)propyl]-L-prolinamide hydrochloride;

N-[4-[N-methylamino]piperidine-1-carbonyl]-L-valyl-N-[2,3-dioxo-3-((1-methyl)ethoxy)-1(S)-(phenylmethyl)propyl]-L-prolinamide hydrochloride;

N-(4-oxopiperidine-1-carbonyl)-L-phenylalanyl-N-[2,3-dioxo-3-((1-methyl)ethoxy)-1-(S)-(phenylmethyl)propyl]-L-prolinamide; and

N-[4-[N-methylamino]piperidine-1-carbonyl]-L-phenylalanyl-N-[3,3,3-trifluoro-2-oxo-1(S)-(phenylmethyl)propyl]-L-prolinamide.

WO96/04248 (corresponding EP 721944 and US 5,691,335) discloses an imidazolidine derivative represented by the formula (IV):

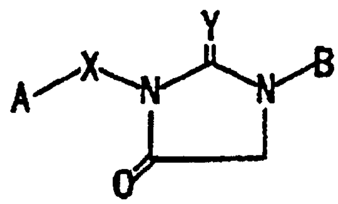

(IV)

(A and B each represents an aromatic hydrocarbon group, which may be substituted with 1-3 groups selected from among halogen, $C_{1-4}$ alkyl, $C_{1-4}$ alkoxy, $C_{1-4}$ alkylenedioxy, phenoxy, nitro, cyano, phenyl, $C_{2-5}$ alkanoylamino, carboxy optionally esterified with a $C_{1-4}$ alkyl or alkenyl group, carboxyalkyl optionally esterified with a $C_{1-4}$ alkyl or alkenyl group; carboxyalkyloxy optionally esterified with a $C_{1-4}$ alkyl or alkenyl group, N-alkylpiperazinylcarbonyl, N-alkylpiperazinylcarbonylalkyl, N-alkylpiperazinylcarbonylalkyloxy, and morpholinocarbonyl; X represents sulfonyl or carbonyl; Y represents oxygen or sulfur). The more preferred are compounds of the above formula wherein A is an aromatic hydrocarbon group which may be substituted with halogen, particularly chlorophenyl; B is an aromatic hydrocarbon group, particularly phenyl; X is sulfonyl; Y is oxygen. As specific examples of the compound represented by the above formula (IV), the following are mentioned.

3-(3-Methoxycarbonylbenzenesulfonyl)-1-phenylimidazolidine-2,4-dione,
3-(4-chlorobenzenesulfonyl)-1-phenylimidazolidine-2,4-dione,
3-(3,4-dimethylbenzenesulfonyl)-1-(3,4-dimethylphenyl)-imidazolidine-2,4-dione,
3-(4-allyloxycarbonylbenzenesulfonyl)-1-(3,4-dimethylphenyl)-imidazolidine-2,4-dione, and
1-(3,4-dichlorophenyl)-3-(2-naphthylsulfonyl)imidazolidine-2,4-dione.

JP Kokai H09-31061 discloses a hydantoin derivative represented by the formula (V):

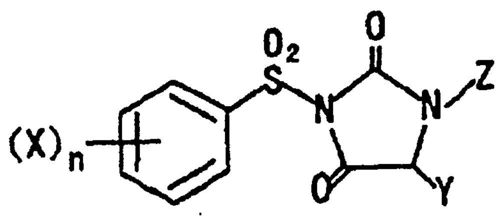

(V)

(wherein X represents halogen, a lower alkyl group of 1-4 carbon atoms, a lower alkoxy group of 1-4 carbon atoms, a carboxyl group which may be esterified with a lower alkyl group of 1-4 carbon atoms or an allyl group; n represents an integer of 0-5; Y represents the group (a)

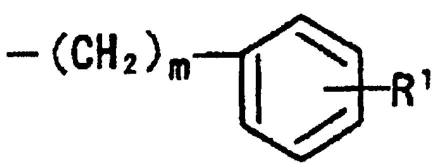

(a)

(wherein $R^1$ represents hydrogen, halogen, hydroxyl, or lower ($C_{1-4}$)alkoxy; m represents 0, 1, or 2), the group (b):

$$-(CH_2)_p-OR^2 \qquad (b)$$

(wherein $R^2$ represents hydrogen or lower($C_{1-4}$)alkyl; p represents 1 or 2), or the group (c):

$$-(CH_2)_q-COOR^3 \qquad (c)$$

(wherein $R^3$ represents hydrogen or lower($C_{1-4}$)alkyl; q represents 1 or 2); Z represents hydrogen, a lower alkyl group of 1-4 carbon atoms, an aralkyl group of 7-10 carbon atoms, or a lower alkyloxycarbonyl group of 2-5 carbon atoms). As specific examples thereof, there are mentioned:

(5R)-5-benzyl-3-(4-chlorobenzenesulfonyl)imidazolidine-2,4-dione and
(5R)-5-benzyl-3-(3,4-dimethylbenzenesulfonyl)imidazolidine-2,4-dione.

WO97/11941 (corresponding EP 795548 and US 5,814,631) discloses a quinazoline derivative represented by the formula (VI):

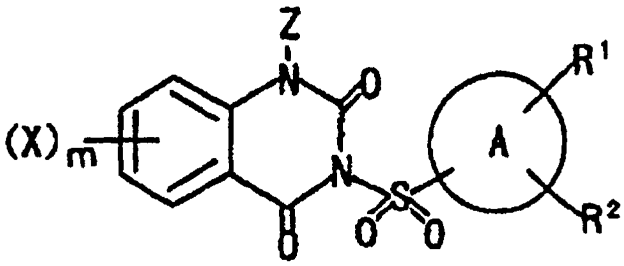

(VI)

(wherein ring A indicates a benzene ring, a pyridine ring, a pyrrole ring, or a pyrazole ring; m represents 0, 1, or 2; X represents hydroxyl, nitro, halogen, lower($C_{1-4}$)alkyl which may be substituted with halogen, lower($C_{1-4}$)alkoxy which may be substituted with halogen, or $C_{7-12}$ aralkyloxy, or X represents a group which, taken together with a benzene ring substituted thereby, forms a naphthalene ring or a quinoline ring; $R^1$ and $R^2$ may be the same or different and each represents hydrogen, halogen, lower($C_{1-4}$)alkyl which may be substituted with halogen, nitro, cyano, pyrazolyl, tetrazolyl, carboxy which may be esterified with lower ($C_{1-4}$)alkyl or allyl, and lower($C_{1-4}$)alkoxy which may be substituted with 1 or more substituents selected from the group consisting of halogen, morpholino, phenylpiperazinyl, and carboxy which may be esterified with lower($C_{1-4}$)alkyl or allyl, or when ring A is a benzene ring, $R^1$ and $R^2$ represent groups which, taken together with the benzene ring substituted thereby, form a naphthalene ring or a quinoline ring; Z represents hydrogen, lower($C_{1-4}$)alkyl which may be substituted with halogen, $C_{2-5}$ alkenyl, aralkyl which may be substituted, aromatic heterocycle-alkyl which may be substituted, carboxymethyl which may be esterified with lower($C_{1-4}$) alkyl or allyl, carbonylmethyl which may be amidated with a primary or secondary amine or a cyclic amine, arylcarbonylmethyl which may be substituted, or aralkyloxymethyl which may be substituted). As specific examples thereof, the following are mentioned.

[7-Chloro-3-(4-chlorobenzenesulfonyl)-2,4(1H,3H)-quinazolinedion-1-yl]acetanilide,
[7-chloro-3-(4-chlorobenzenesulfonyl)-2,4(1H,3H)-quinazolinedion-1-yl]acetic 4-hydroxyanilide,
[7-chloro-3-(4-chlorobenzenesulfonyl)-2,4(1H,3H)-quinazolinedion-1-yl]acetic 3-pyridineamide,
[7-chloro-3-(4-chlorobenzenesulfonyl)-2,4(1H, 3H)-quinazolinedion-1-yl]acetic 4-pyridineamide,
[7-chloro-3-(4-chlorobenzenesulfonyl)-2,4(1H, 3H)-quinazolinedion-1-yl]acetic 2-pyridineamide,
3-{[7-chloro-3-(4-chlorobenzenesulfonyl)-2,4(1H,3H)-quinazolinedion-1-yl]acetylamino}-N-ethylpyridinium iodide,
[7-chloro-3-(4-chlorobenzenesulfonyl)-2,4(1H,3H)-quinazolinedion-1-yl]acetic 5-indoleamide,
3-{[7-chloro-3-(4-chlorobenzenesulfonyl)-2,4(1H,3H)-quinazolinedion-1-yl]acetylamino}-(N-t-butoxycarbonylmethylpyridinium) bromide,
3-{[7-chloro-3-(4-chlorobenzenesulfonyl)-2,4(1H,3H)-quinazolinedion-1-yl]acetylamino}-N-carboxymethylpyridinium bromide,
3-[7-chloro-3-(4-chlorobenzenesulfonyl)-2,4(1H,3H)-quinazolinedion-1-yl]acetylaminophenoxyphosphoric acid,
7-chloro-3-(4-chlorobenzenesulfonyl)-1-(3-pyridylmethyl)-2,4(1H,3H)-quinazolinedione, and
[7-chloro-3-(4-chlorobenzenesulfonyl)-2,4(1H,3H)-quinazolinedion-1-yl]acetic 4-chloroanilide.

EP 0713876 discloses a compound represented by the formula (VIIa) or (VIIb):

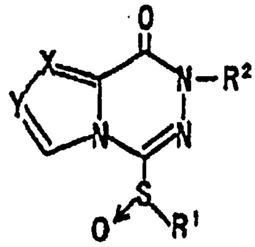

(VIIa)

or

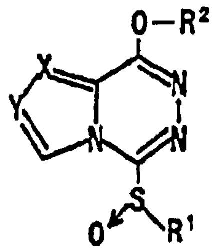

(VIIb)

[In formula (VIIa), in cases where X represents C-$CH_3$ and Y represents N, $R^1$ represents a lower alkyl group or a benzyl group substituted by one halogen atom and $R^2$ represents a lower alkyl group, a benzyl group substituted by one halogen atom, or a lower alkoxycarbonylmethyl group. In formula (VIIb), in cases where X represents N and Y represents CH, where X represents CH and Y represents CH, and where X represents C-$CH_3$ and Y represents N, $R^1$ represents a lower alkyl group, a lower alkoxycarbonylmethyl group, a phenyl(lower)alkyl group, or a benzyl group nuclearly substituted by any one of lower alkyl, halogen, cyano, phenyl and halo(lower)alkyl, and $R^2$ represents hydrogen, a lower alkyl group, a lower alkoxycarbonylmethyl group, a phenyl(lower)alkyl group, or a benzyl group nuclearly substituted by any one of lower alkyl, halogen, cyano, phenyl, and halo(lower)alkyl] and, as specific examples, mentions the following compounds:

5-(4-Chlorobenzylsulfinyl)-8-hydroxyimidazo[1,2-d][1,2,4]triazine,
8-(4-chlorobenzyloxy)-5-(4-chlorobenzylsulfinyl)imidazo[1,2-d][1,2,4]triazine,
8-(4-methylbenzyloxy)-5-(4-methylbenzylsulfinyl)imidazo[1,2-d][1,2,4]triazine,
8-(3-chlorobenzyloxy)-5-(3-chlorobenzylsulfinyl)imidazo [1,2-d][1,2,4]triazine,
1-(4-chlorobenzyloxy)-4-(4-chlorobenzylsulfinyl)-8-methylimidazo[1,5-d][1,2,4]triazine,
2-(4-chlorobenzyl)-4-(4-chlorobenzylsulfinyl)-8-methylimidazo [1,5-d][1,2,4]triazin-1(2H)-one,
5-(4-chlorobenzylsulfinyl)-8-ethoxycarbonylmethyloxyimidazo [1,2 d][1,2,4]triazine,
4-(4-chlorobenzylsulfinyl)-1-(ethoxycarbonylmethyloxy)-8-methylimidazo[1,5-d][1,2,4]triazine, and
2-ethoxycarbonylmethyl-4-(4-chlorobenzylsulfinyl)-8-methylimidazo[1,5-d][1,2,4]triazin-1(2H)-one.

JP Kokai S10-87567 discloses a compound, inclusive of a salt thereof, which is represented by the formula (VIII):

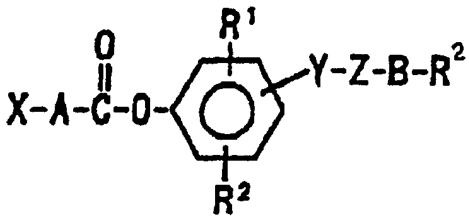

(VIII)

[wherein $R^1$ and $R^2$ may be the same or different and each represents a hydrogen atom, a hydroxyl group, or a lower alkyl or lower alkoxy group which may optionally be substituted; X represents a carbocyclic or heterocyclic group which may optionally be substituted; A represents a bond or a lower alkylene or imino group which may optionally be substituted; Y represents a carbonyl group, a sulfonyl group, or a lower alkylene or imino group which may optionally be substituted; Z represents a phenylene, bivalent heterocyclic, or imino group which may optionally be substituted; B represents a bond, a lower alkylene group, or a phenylene group; $R^3$ represents an acyl group or a carboxyl group which may optionally be esterified or amidated], and as specific examples, the following are mentioned.

1-[4-(3-Indolylacetoxy)benzoyl-4-piperidinecarboxylic acid,

(S)-2-[4-(3-indolylacetoxy)benzoyl]-1,2,3-4-tetrahydroisoquinoline-3-carboxylic acid,
(S)-1-[4-(3-indolylacetoxy)benzoyl]pyrrolidine-2-carboxylic acid,
(S)-1-[4-[3-(2-methyl)indolylacetoxy]benzoyl]pyrrolidine-2-carboxylic acid

WO96/33974 (corresponding EP 826671 and US 5,948,785) discloses a heterocyclic amide compound, inclusive of a pharmacologically acceptable salt thereof, which is represented by the formula (IX)

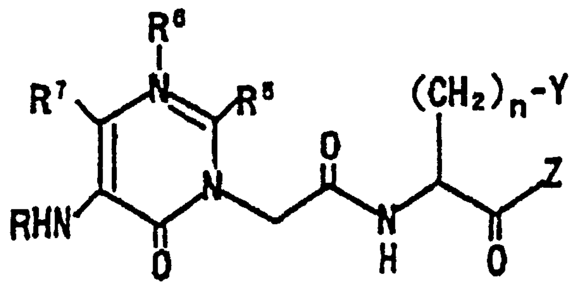

(IX)

[wherein R represents hydrogen, -CHO-, $-CONH_2$, $-COR^1$, $-COOR^1$-, $-CONHOR^1$, $-CONHR^1$, $-CONR^1R^{1'}$, $-CONHSO_2R^1$, $-COSR^1$, $-COCOR^2$, $-COCOOR^2$, $-CONHCOOR^2$, $-COCONR^3R^4$, $-CSXR^1$, $-SO_2WR^1$, $-SO_2NR^1R^{1'}$ or $-SO_2E$ (in the above formulas, $R^1$ and $R^{1'}$ may be the same or different and each independently represents alkyl, cycloalkyl, cycloalkylalkyl, aryl, arylalkyl, heteroaryl, heteroarylalkyl, heterocycle or heterocycle-alkyl; $R^2$, $R^3$ and $R^4$ may be the same or different and each independently represents hydrogen, alkyl, or arylalkyl; or $-NR^3R^4$ may as a unit represent a heterocycle; X represents a direct bond, -NH-, -O-, or -S-; W represents a direct bond, -NH-, -NHCO-, -NHCOO-, or -NHCONH-; E represents hydroxyl or amino); $R^5$, $R^6$ and $R^7$ may be the same or different and each independently represents hydrogen or alkyl, or one of $R^5$, $R^6$, and $R^7$ represents aryl, arylalkyl, arylalkenyl, heteroaryl, heteroarylalkyl, or heteroarylalkenyl, while the others each represents hydrogen; M represents carbon or nitrogen, with the proviso that when M is nitrogen, $R^6$ does not exist; Y represents cycloalkyl, aryl, or heteroaryl; Z represents $-CF_2R^8$, $-CF_2CONR^9R^{10}$, $-CF_2COOR^9$, $-COOR^9$, or $-CONR^9R^{10}$ ($R^8$ represents hydrogen, halogen, alkyl, perfluoroalkyl, aminoalkyl, alkylaminoalkyl, dialkylaminoalkyl, alkoxyalkyl, hydroxyalkyl, aryl, arylalkyl, arylalkenyl, heteroaryl, heteroarylalkyl, or heteroarylalkenyl; $R^9$ and $R^{10}$ may be the same or different and each independently represents hydrogen, alkyl, alkenyl, cycloalkyl, cycloalkylalkyl, heterocycle-alkyl, aryl, arylalkyl, arylalkenyl, heteroaryl, heteroarylalkyl, or heteroarylalkenyl; $-NR^9$, $R^{10}$ may jointly represent a heterocycle); n represents 0 or 1; it is to be noted that, among the above-mentioned groups, the alkyl, cycloalkyl, cycloalkylalkyl, aryl, arylalkyl, arylalkenyl, heteroaryl, heteroarylalkyl, heteroarylalkenyl, heterocycle, and heterocycle-alkyl may each have a substituent or substituents]. As specific examples of the above compound, the following are mentioned.

2-[5-Amino-2-(3,5-diaminophenyl)-6-oxo-1,6-dihydro-1-pyrimidyl]-N-(1-benzyl-3,3,3-trifluoro-2-oxopropyl)acetamide and
2-(5-hydroxysuccinylamino-6-oxo-2-phenyl-1,6-dihydro-1-pyrimidyl)-N-(1-benzyl-3,3,3-trifluoro-2-oxopropyl) acetamide.

JP Kokai H09-124691 discloses a peptide compound, inclusive of a pharmacologically acceptable salt thereof, which is represented by the formula (X):

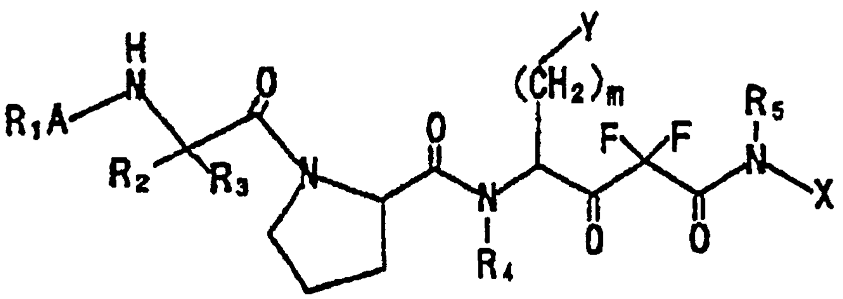

(X)

[wherein X represents a group:

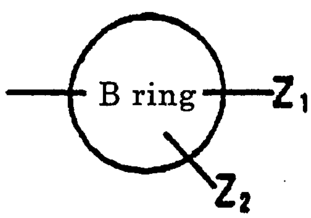

or -CH-$R_6$ $R_7$
(where $Z_1$ and $Z_2$ may be the same or different and each represents hydrogen, a hydroxyl group, a group represented by -$COR_8$, an alkyl group which may optionally be substituted by a group represented by -$COR_8$, or a group represented by -$SO_2R_8$; $R_6$ and $R_7$ may be the same or different and each represents hydrogen, cycloalkyl, cycloalkylalkyl, aryl, arylalkyl, heteroaryl, heteroarylalkyl, a group represented by -$COR_8$, or an alkyl, alkenyl, alkynyl, heterocycle or heterocyclealkyl group substituted by a group represented by -$COR_8$; provided, however, that $R_6$ and $R_7$ do not concurrently represent hydrogen and that in cases where either $R_6$ or $R_7$ or both represent heterocycles substituted by a group represented by -$COR_8$, the respective heterocycles are not bound through any hetero atom thereof to the carbon atom attached to $R_6$ and $R_7$, or $R_6$ and $R_7$ may jointly represent a $C_{4-6}$ methylene chain which may optionally be substituted by a group represented by -$COR_8$; B ring stands for an aromatic ring);

$R_1$ represents an alkyl, cycloalkyl, cycloalkylalkyl, alkoxy, aryl, arylalkyl, heteroaryl, heterocycle, or heterocyclealkyl group which may optionally be substituted by a group represented by -$COR_8$;
$R_2$ and $R_3$ may be the same or different and each represents hydrogen or an alkyl, cycloalkyl, cycloalkylalkyl, aryl or arylalkyl group which may optionally be substituted by a group represented by -$COR_8$, or $R_2$ and $R_3$ may jointly represent a $C_{4-6}$ methylene chain which may optionally be substituted by a group represented by -$COR_8$;
$R_4$ represents hydrogen or an alkyl group;
$R_5$ represents hydrogen or an alkyl group; or in the case where X represents -CH-$R_6$ $R_7$, $R_5$ and $R_6$ may jointly represent a $C_{2-4}$ methylene chain;
$R_8$ represents a hydroxy, alkoxy, amino, alkylamino, dialkylamino, aryloxy, or arylalkoxy group; Y represents a cycloalkyl, aryl or heteroaryl group;
m represents an integer of 0 or 1-3, and A represents a carbonyl or sulfonyl group], and as specific examples, the following compounds are mentioned.
N-[4(S)-[N-[N-(tert-butyloxycarbonyl)-L-valyl-L-prolyl] amino]-2,2-difluoro-3-oxo-5-phenylvaleryl]glycine,
3-N-[4(S)-[N-[N-(tert-butyloxycarbonyl)-L-glutamyl-L-prolyl]amino]-2,2-difluoro-3-oxo-5-phenylvaleryl]amino]benzoic acid, and
3-[N-[4(S)-[N-[N-(phenylsulfonyl)-L-glutamyl-L-prolyl]amino]-2,2-difluoro-3-oxo-5-phenylvaleryl]amino]benzoic acid

JP Kokai H10-7661 describes a heterocyclic amide compound, inclusive of a pharmacologically acceptable salt thereof, which is represented by the formula (XI):

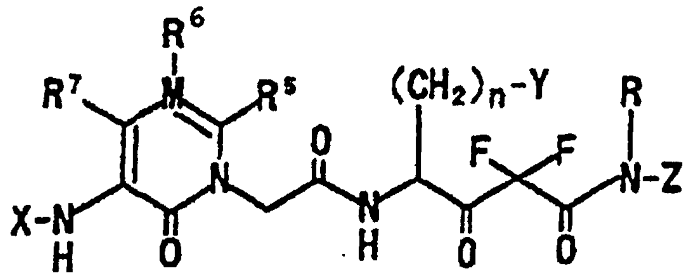

(XI)

[wherein X represents -$COOR^1$, -$(CH_2)_mCOOR^1$, or -$CO(CH_2)_mCOOR^1$ ($R^1$ represents hydrogen, alkyl, cycloalkyl, cycloalkylalkyl, aryl, arylalkyl, heteroaryl, heteroarylalkyl, heterocycle, or heterocyclealkyl; m represents 1, 2, or 3); Z represents - $(CH_2)_rCOOR^2$ or a group of the formula (i):

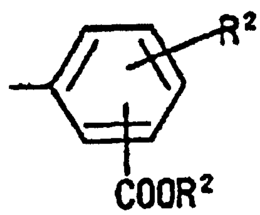

(i)

(wherein $R^2$ represents hydrogen, alkyl, cycloalkyl, cycloalkylalkyl, aryl, arylalkyl, heteroarylalkyl, heterocycle, or heterocyclealkyl; $R^3$ represents hydrogen, alkyl, alkoxy, or halogen; r represents 1, 2, or 3); R represents hydrogen or alkyl; $R^5$, $R^6$ and $R^7$ may be the same or different and each independently represents hydrogen or alkyl, or one of $R^5$, $R^6$ and $R^7$ represents aryl, arylalkyl, arylalkenyl, heteroaryl, heteroarylalkyl or heteroarylalkenyl, while the remaining others each represents hydrogen; M represents carbon or nitrogen; provided, however, that in the case where M is nitrogen, $R^6$ does not exist; Y represents cycloalkyl, aryl, or heteroaryl; n represents 0 or 1; among the above groups, said aryl, arylalkyl, heteroaryl and heteroarylalkyl may each be substituted]. The preferred is the compound in which X is -$CO(CH_2)_mCOOH$ (m is 1, 2, or 3, more preferably 2); Y is aryl, more preferably phenyl; Z is -$CO(CH_2)_rCOOH$ (r is 1, 2, or 3, more preferably 1); M is nitrogen, $R^5$ is aryl which may optionally be substituted by alkyl, more preferably methyl, particularly preferably phenyl; and $R^7$ is hydrogen. Specific examples of the compound represented by the formula (IX) are:

2-[1,6-dihydro-5-hydroxysuccinylamino-2-(3-methylphenyl)-6-oxo-1-pyrimidinyl]-N-[1-benzyl-3,3-difluoro-2-oxo-3-[N-(carboxymethyl)carbamoyl]propyl]acetamide and
2-[1,6-dihydro-5-hydroxysuccinylamino-2-(3-methylphenyl)-6-oxo-1-pyrimidinyl]-N-[1-benzyl-3,3-difluoro-2-oxo-3-[N-(3-carboxyphenyl)carbamoyl]propyl]acetamide.

JP Kokai H10-53579 discloses a compound, inclusive of a salt thereof, which is represented by the formula (XII):

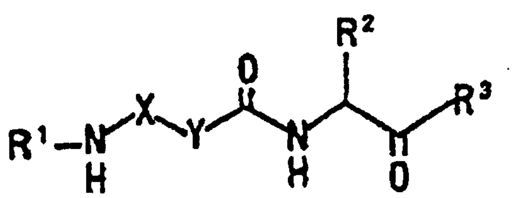

(XII)

[wherein $R^1$ represents hydrogen or an amino-protecting group; $R^2$ represents an ar(lower)alkyl group; $R^3$ represents a lower alkyl group optionally substituted by one or more halogen atoms, a carboxyl group, or a protected carboxyl group; X represents a group of the formula;

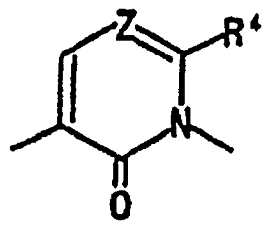

or

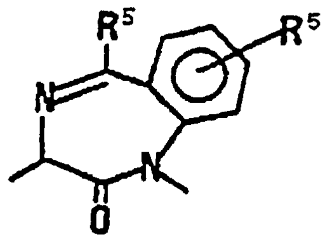

(wherein $R^4$ or $R^5$ represents an aryl group optionally substituted by not less than one halogen, lower alkoxy, or phenyl; or a cyclo(lower)alkyl group; $R^6$ represents hydrogen or a lower alkyl group; Z represents -N= or -CH=); Y represents a lower alkylene group]. As specific compounds, there are mentioned:

2-[5-[(benzyloxycarbonyl)amino]-2-(4-fluorophenyl)-1,6-dihydro-6-oxo-1-pyrimidinyl]-N-[2-(4,4,4-trifluoro-3-oxo-1-phenyl)butyl]acetamide,
2-[5-amino-2-(4-fluorophenyl)-1,6-dihydro-6-oxo-1-pyrimidinyl]-N-[2-(4,4,4-trifluoro-3-oxo-1-phenyl)butyl]acetamide,
2-[5-amino-2-phenyl-1,6-dihydro-6-oxo-1-pyrimidinyl]-N-[2-(4,4,4-trifluoro-3-oxo-1-phenyl)butyl]acetamide,
2-(5-amino-1,6-dihydro-6-oxo-2-phenyl-1-pyrimidinyl)-N-[2-(3-methoxycarbonyl-3-oxo-1-phenyl)butyl]acetamide, and
2-(3-amino-2,3-dihydro-2-oxo-5-phenyl-1H-1,4-benzodiazepin-1-yl)-N-[2-(4,4,4-trifluoro-3-oxo-1-phenyl)butyl] acetamide.

WO98/09949 (corresponding EP 936216) discloses an acetamide derivative represented by the formula (XIII):

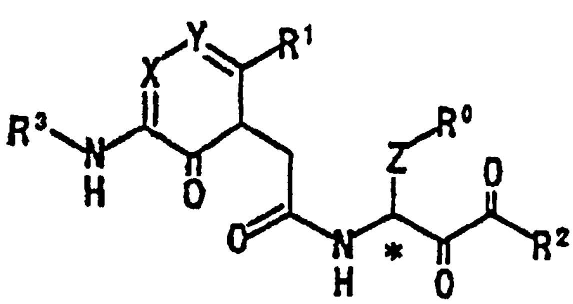

(XIII)

[wherein $R^0$ represents a phenyl group which may have one or more substituents on the ring as selected from Group A defined below (Group A: Group A represents halogen, nitro, hydroxyl, lower alkoxy, lower alkyl, or halogen-substituted

lower alkyl);

$R^1$ represents (i) aryl, (ii) heteroaryl, or (ii) $C_{1-6}$ straight-chain, branched-chain or cyclic alkyl, which may each independently have one or more substituents defined above as Group A; or $R^1$ may have one or more substituents selected from Group B consisting of $OR_a$, $COOR_a$, $CONR_bR_c$, $NR_bR_c$, $NR_bCHO$, $NR_bCOR_a$, $SO_2OR_a$, $SO_2R_a$, $CONR_bSO_2R_a$, and $P(O)(OR_a)_2$ on any of said groups (i)~(iii) (in the above formulas, $R_a$~$R_c$ independently represent hydrogen, lower alkyl or substituted lower alkyl; or $R_a$~$R_c$ each independently is aryl($C_{1-7}$)alkyl, heteroaryl($C_{1-7}$)alkyl, aryl, or heteroaryl, where the aryl or heteroaryl may nuclearly have one or more, usually 1-3, substituents selected from Group A defined above. The substituted lower alkyl has 1-3 atoms or groups selected from among halogen, nitro, and hydroxyl); or $R^1$ may have one or more members of Cyclic Group G defined below as substituents on any of said groups (i)~(iii)(Cyclic Group B: Cyclic Group G represents a 5-membered or 6-membered heterocyclic group containing oxygen or nitrogen within the range of 1-3 atoms and may have a substituent or substituents).

$R^2$ represents ($C_{1-8}$)alkyl, aryl($C_{1-7}$)alkyl, heteroaryl($C_{1-7}$)alkyl or aryl; or $R^2$ represents Group B defined above or a ($C_{1-8}$)alkyl group having group B as a substituent; or a ($C_{1-8}$)alkyl group having the above-defined Cyclic Group G as a substituent.

$R^3$ is hydrogen; or $R^3$ may represent an acyl group which is (i) $D(CH_2)_{0-3}\cdot CO$, (ii) $D\cdot CO\cdot E\cdot CO$, or (iii) $D\cdot SO_2\cdot E\cdot CO$ or $R^3$ represents a sulfonyl group which is $D(CH_2)_{0-3}\cdot SO_2$ or $D\cdot CO\cdot E\cdot SO_2$ (where group D is hydrogen, $C_{1-6}$ straight-chain, branched-chain, or cyclic alkyl, aryl, halo(lower)alkyl, halo(lower)alkoxy, amino, lower alkoxyamino, halo(lower)alkylamino, $R_bR_cN$, $R_bR_cNO$, $R_aO$, $R_a$, $R_aOCO$, $R_bR_cNCO$, $R_aSO_2NR_b$, $R_aS$, or Cyclic group G defined above. Group E represents a bivalent bridging group of 1-6 carbon atoms); or $R^3$ is a urea group represented by $R_bR_cNCO$; or $R^3$ is a thiourea represented by $R_bR_cN\cdot CS$; or $R^3$ is $R_a$.

X and Y each independently represents nitrogen or carbon, which may optionally be substituted by the groups represented by $R_a$~$R_c$.

Z represents a polymethylene group and the hydrogen atoms on the polymethylene group may each independently be substituted with $R_a$ or $R_b$]. As specific examples, the following compounds are mentioned.

2-(5-Amino-6-oxo-2-phenyl-1,6-dihydropyrimidin-1-yl)-N- [2,3-dioxo-1-phenylmethyl-6-(2-pyridyloxy)]hexylacetamide 2HCl,
2-[6-oxo-2-phenyl-5-(4-pyridylmethyloxycarbonylamino)-1,6-dihydropyrimidin-1-yl]-N-[2,3-dioxo-1-phenylmethyl-6-(2-pyridyloxy)]hexylacetamide,
2-(5-formylamino-6-oxo-2-phenyl-1,6-dihydropyrimidin-1-yl)-N-[2,3-dioxo-1-phenylmethyl-6-(2-pyridyloxy)]hexylacetamide, and
2-(5-benzylaminosulfonylamino-6-oxo-2-phenyl-1,6-dihydropyrimidin-1-yl)-N-[2,3-dioxo-1-phenylmethyl-6-(2-pyridyloxy)]hexylacetamide.

WO98/18794 (corresponding EP 940400 and US 6,080,738) discloses a heterocyclic amide compound, inclusive of a pharmacologically acceptable salt thereof, which is represented by the formula (XIV):

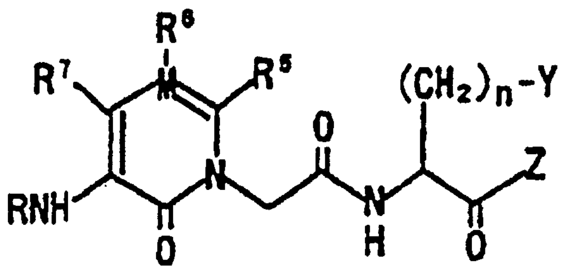

(XIV)

[wherein R represents hydrogen, alkyl, -COH-, $-CONH_2$, $-COR^1$, $-COOR^1$, $-CONHOR^1$, $-CONHR^1$, $-CONR^1R^{1'}$, $-CONHSO_2R^1$, $-COSR^1$, $-COCOR^2$, $-COCOOR^2$, $-CONHCOOR^2$, $-COCONR^3R^4$, $-CSXR^1$, $-SO_2WR^1$, $-SO_2NR^1R^{1'}$, or $-SO_2E$ (in the above formulas, $R^1$ and $R^{1'}$ may be the same or different and each independently represents alkyl, cycloalkyl, cycloalkylalkyl, aryl, arylalkyl, heteroaryl, heteroarylalkyl, heterocycle, or heterocycle-alkyl; $R^2$, $R^3$, and $R^4$ may be the same or different and each independently represents hydrogen, alkyl, or arylalkyl; or $-NR^3R^4$ may as a unit represent a heterocycle; X represents a single bond, -NH-, -O-, or -S-; W represents a single bond, -NH-, -NHCO-, -NHCOO-, or -NHCONH-; E represents hydroxyl or amino); $R^5$, $R^6$, $R^7$ may be the same or different and each independently represents hydrogen or alkyl, or one of $R^5$, $R^6$, $R^7$ represents aryl, arylalkyl, arylalkenyl, heteroaryl, heteroarylalkyl, or heteroarylalkenyl, with the remaining others each representing hydrogen; M represents carbon or nitrogen, with the proviso that when M is nitrogen, $R^6$ does not exist; Y represents cycloalkyl, aryl, or heteroaryl; Z represents a group of the formula (i),

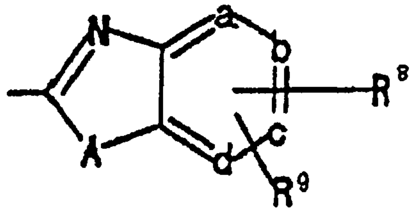

(i)

the formula (ii)

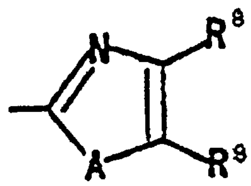

(ii)

or the formula (iii)

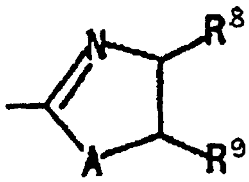

(iii)

[wherein $R^8$ and $R^9$ may be the same or different and each independently represents hydrogen, alkyl, aryl, arylalkyl, heteroaryl, heteroarylalkyl, halogen, trifluoromethyl, cyano, nitro, $-NR^{10}R^{10'}$, $-NHSO_2R^{10}$, $-OR^{10}$, $-COOR^{10}$, $-CONHSO_2R^{10}$ or $-CONR^{10}R^{10'}$ ($R^{10}$ and $R^{10'}$ may be the same or different and each independently represents hydrogen, alkyl, cycloalkyl, cycloalkylalkyl, aryl, arylalkyl, heteroaryl, heteroarylalkyl, or trifluoromethyl; $-NR^{10}R^{10'}$ may as a unit represent a heterocycle); A represents -O-, -S-, or $-NR^{12}-$ ($R^{12}$ represents hydrogen, alkyl, cycloalkyl, or cycloalkylalkyl); a, b, c, and d each represents carbon or any one of them is nitrogen, with the remaining others each representing carbon]; n represents 0 or 1.

Among the above groups, the alkyl, cycloalkyl, cycloalkylalkyl, aryl, arylalkyl, arylalkenyl, heteroaryl, heteroarylalkyl, heteroarylalkenyl, heterocycle, and heterocyclealkyl may each have a substitutent or substituents.]

Furthermore, JP Kokai H10-245384 discloses a triazinesulfonic acid derivative represented by the formula (XV) or (XVI):

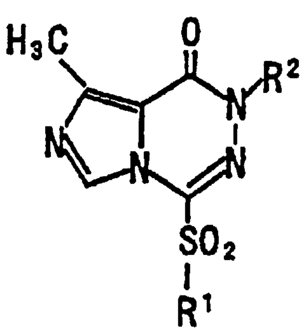

(XV)

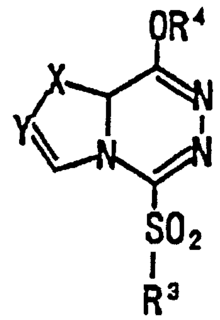

(XVI)

[In formula (XV), $R^1$ represents a lower alkyl group or a benzyl group substituted by one halogen atom. $R^2$ represents a lower alkyl group, a benzyl group substituted by one halogen atom, or a lower alkoxycarbonylmethyl group. In formula (XVI), X and Y each represents a combination of N and CH, CH and CH, or C-$CH_3$ and N. $R^3$ represents a lower alkyl group, a lower alkoxycarbonylmethyl group, a phenyl-lower alkyl group, or a benzyl group nuclearly substituted by lower alkyl or halogen. $R^4$ represents hydrogen, a lower alkyl group, a lower alkoxycarbonylmethyl group, a phenyl (lower)alkyl group, or a benzyl group nuclearly substituted by lower alkyl or halogen]. The specific relevant compounds mentioned are:

8-(4-chlorobenzyloxy)-5-(4-chlorobenzylsulfonyl)imidazo[1.2-d][1,2,4]triazine,
8-(4-methylbenzyloxy)-5-(4-methylbenzylsulfonyl)-imidazo[1.2-d][1,2,4]triazine,
8-pentyloxy-5-pentylsulfonylimidazo[1.2-d][1,2,4]triazine,
5-(4-chlorobenzylsulfonyl)-8-ethoxycarbonylmethyloxyimidazo[1.2-d][1,2,4]triazine,
5-(4-(chlorobenzylsulfonyl)-8-hydroxyimidazo [1.2-d][1,2,4]triazine,
8-phenethyloxy-5-phenethylsulfonylimidazo[1.2-d] [1,2,4]triazine,
8-ethoxycarbonylmethyloxy-5-ethoxycarbonylmethylsulfonylimidazo[1.2-d][1,2,4]triazine.

In the present invention, chymostatin and said compounds of formulas (I)~(XVI), inclusive of their salts, among others, can be used as the active ingredient. As the salts, pharmaceutically or pharmacologically acceptable salts are used. For example, there can be mentioned alkali metal salts such as sodium salt, potassium salt, etc.; alkaline earth metal salts such as calcium salt, magnesium salt, etc.; salts with inorganic bases, such as aluminum salt, ammonium salt, etc.; salts with organic bases such as trimethylamine, pyridine, picoline, ethanolamine, diethanolamine, triethanolamine, dicyclohexylamine, N,N-dibenzylethylenediamine, etc.; salts with inorganic acids such as hydrochloric acid, hydrobromic acid, nitric acid, sulfuric acid, phosphoric acid, etc.; salts with organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; and salts with amino acids such as arginine, lysine, ornithine, aspartic acid, glutamic acid, and so forth.

The ciliary muscle relaxant of the invention may be formulated together with a pharmaceutically acceptable carrier or excipient and processed into per se known dosage forms by the known pharmaceutical procedure, for example by blending and/or dissolving the ingredients. Among such dosage forms, the dosage form for oral administration to the human includes but is not limited to powders, granules, tablets, capsules, syrups, and solutions. Formulations for powders, granules, tablets or the like may include various pharmaceutical additives suitable for solid preparations, for example an excipient (e.g. starch, glucose, fructose, sucrose, etc.), a lubricant (magnesium stearate etc.), a disintegrator (starch, crystalline cellulose, etc.), and a binder (starch, gum Arabic, etc.), among others. These preparations may be coated with a coating agent (e.g. gelatin, sucrose, or the like) or an enteric film coating base (e.g. hydroxypropylmethylcellulose phthalate, a methacrylic copolymer, a ceramic coating, or the like) so that the prearations will be selectively dissolved in the intestinal tract. When the intended dosage form is syrup or solution, such formulating additives as a stabilizer (sodium edetate etc.), a suspending agent (gum Arabic, carmellose, etc.), a corrigent (simple syrup, glucose, etc.), a flavorant, etc. can be selectively employed. The dosage form for parenteral administration includes injections and suppositories. When the intended dosage form is an injection, such formulating additives as a solvent (distilled water for injection etc.), a stabilizer (sodium edetate etc.), an isotonizing agent (sodium chloride, glycerin, mannitol, etc.), a pH control agent (hydrochloric acid, citric acid, sodium hydroxide, etc.), and a suspending agent (methylcellulose etc.) can be employed.

The ophthalmic topical dosage form includes water-based eyedrops including ophthalmic aqueous suspensions, solutions, gels, emulsions, and the like. Non-aqueous eyedrops such as oily eyedrops, ophthalmic ointments, and sustained- or controlled-release eyedrops may also be available choices.

In preparing eyedrops, such known formulating additives as a solvent (physiological saline, purified water, etc.), a chelating agent (sodium edetate, citric acid, etc.), an antioxidant (sulfurous acid, a salt thereof, etc.), an emulsifier (polyvinylpyrrolidone etc.), a suspending agent and/or a thickener (macromolecular compounds, e.g. cellulose derivatives such as hydroxypropylmethylcellulose, methylcellulose, etc., polyvinyl alcohol, polyvinylpyrrolidone, carboxyvinyl polymer, and so on), a surfactant (e.g. nonionic surfactants such as polysorbate 80, polyoxyethylene-hydrogenated castor oil, etc.), a preservative (quaternary ammonium salts such as benzalkonium chloride, benzetonium chloride, etc., parabens, chlorobutanol, and so on), a buffer (boric acid, borax, sodium acetate, citrate buffer, phosphate buffer, etc.), an isotonizing agent (sodium chloride, glycerol, mannitol, sorbitol, etc.), a pH control agent (hydrochloric acid, sodium hydroxide, etc.) can be selectively employed. Ophthalmic ointments may be prepared using ointment bases such as white petrolatum, lanolin and so forth.

The ciliary muscle relaxant of the present invention is useful for the prevention or treatment of myopia. Myopia, referred to here, includes pseudomyopia, low myopia and high myopia, among others, and the ciliary muscle tension-reducing activity according to the present invention can be exploited with advantage in the preparation of a prophylactic or therapeutic drug for said types of myopia but also effective in asthenopia arising from hypertonia of the ciliary muscle.

The dosage of the ciliary muscle relaxant according to the present invention varies depending on the kind of active ingredient chymase inhibitor, the disease to be dealt with, clinical condition, subject, method of administration, etc. and is not particularly restricted. However, the effective amount per dose of the compound of formula (IV), for instance, or a salt thereof for an adult human is usually 1-1000 mg, preferably 10-500 mg, for oral administration or 0.1-300 mg, preferably 1-150 mg, in the case of an injection. For topical application, eyedrops containing usually 0.001-1.0 w/v%, preferably 0.01-0.5w/v%, of the compound can be instilled into the eye, 20-50 μl per dose about 5-6 times a day, for rewarding results.

Unless contrary to the object of the invention, the ciliary muscle relaxant of the present invention may comprise other medicaments in addition to the chymase inhibitor. As such other medicaments, there can be mentioned anticoagulants such as warfarin potassium, urokinase, aspirin, etc.; vasotonic/hemostatic drugs such as sodium carbazochrome sulfonate etc.; circulation improving agents such as kallidinogenase, pentoxifylline, sarpogrelate hydrochloride, tocopherol nicotinate, etc.; adrenocortical steroids such as betamethasone, dexamethasone, prednisolone, etc.; anti-inflammatory enzymes such as serapeptidase, streptokinase, streptodornase, antiglaucoma drugs such as β-blockers, mannitol, acetazolamide, etc., and prostaglandin antagonists.

BRIEF DESCRIPTION OF THE DRAWINGS

Fig. 1 is a graph showing the relaxing effect of a chymase inhibitor (Compound 1 described hereinafter) on the carbachol-induced contraction of the rabbit ciliary muscle.
Fig. 2 is a graph showing the relaxing effect of a chymase inhibitor (Compound 2 described hereinafter) on the carbachol-induced contraction of the canine ciliary muscle.

On the graph, * denotes a significant difference of Compound 2 ($10^{-5}$ M) against carbachol ($10^{-4}$ M), $p<0.05$ (Student's t-test).

BEST MODE FOR CARRYING OUT THE INVENTION

EXAMPLES

The following examples of synthesis, working examples, and test examples are intended to illustrate the present invention in further detail and should by no means construed as defining the scope of the invention.

Example 1

Tablets

| | |
|---|---|
| 3-(4-Chlorobenzenesulfonyl)-1-phenylimidazolidine-2,4-dione | 200 mg |
| Lactose | 80 mg |
| Starch | 17 mg |
| Magnesium stearate | 3 mg |

Tablets (continued)

| | |
|---|---|
| Crystalline cellulose | 10 mg |

Using the above ingredients as the raw material per tablet, tablets were manufactured by the established procedure. Where necessary, these tablets may be coated with the conventional enteric coating (e.g. hydroxypropylmethylcellulose phthalate), sugar coating, or film coating (e.g. ethylcellulose) composition.

Example 2

| Capsules | |
|---|---|
| 3-(4-Chlorobenzenesulfonyl)-1-phenylimidazolidine-2,4-dione | 100 mg |
| Mannitol | 75 mg |
| Starch | 17 mg |
| Calcium stearate | 3 mg |

The above ingredients, per capsule, were evenly blended, granulated, and filled in a hard capsule shell by the routine procedure. Where necessary, the granules for filling may be coated with the conventional enteric coating (e.g. hydroxypropylmethylcellulose phthalate), sugar coating, or film coating (e.g. ethylcellulose) composition.

Example 3

| Injectable suspension | |
|---|---|
| 3-(4-Chlorobenzenesulfonyl)-1-phenylimidazolidine-2,4-dione | 5 mg |
| Carboxymethylcellulose sodium | 500 mg |
| Distilled water for injection | To make 100 ml |

The above ingredients were aseptically admixed in the routine manner to prepare an injectable suspension.

Example 4

| Eye-drops | |
|---|---|
| 3-(4-Chlorobenzenesulfonyl)-1-phenylimidazolidine-2,4-dione | 1.0 g |
| Sodium dihydrogen phosphate dihydrate | 0.1 g |
| Polysorbate 80 | 0.1 g |
| Sodium chloride | 0.9 g |
| Sodium hydroxide | q.s. |
| Sterilized pure water | To make 100 ml |

The above ingredients were admixed in the routine manner to produce an ophthalmic suspension for instillation.

Test Example 1 (the ciliary muscle relaxant effect of the chymase inhibitor in rabbits)

(1) Experimental animals

Male albino rabbits weighing about 2 kg were used.

(2) Test drugs

Ciliary muscle relaxant: chymase inhibitor
Ciliary muscle constricting agent: carbachol (carbamylcholin chloride, product of Sigma)
As the chymase inhibitor, 3-(4-chlorophenylsulfonyl)-1-phenylimidazolidine (referred to as Compound 1) was dissolved in dimethyl sulfoxide and used.
Carbachol was dissolved in distilled water and used.

(3) Test protocol

The eyeball was enucleated and a 2~3 mm-wide ciliary muscle specimen in radial direction was prepared. The specimen was suspended in a Magnus tube (10 mL) filled with Krebs-Henseleit solution (118 mM NaCl, 4.7 mM KCl, 1.2 mM $CaCl_2$, 1.2 mM $KH_2PO_4$, 1.2 mM $MgSO_4$, 25 mM $NaHCO_3$, 11.5 mM glucose, 37°C, pH 7.4) aerated with 95% $O_2$-5% $CO_2$, and equilibrated for 30 minutes under a static tension of 50 mg. The change in tension was isometrically recorded on a recorder (U-228, manufactured by Nippon Denko Kagaku) through a transducer (TB-612T, manufactured by Nippon Kohden) and an amplifier (SEN-6102, manufactured by Nippon Kohden) to evaluate the muscle relaxant action of the chymase inhibitor on carbachol ($10^{-4}$ M)-induced persistent contraction of the rabbit ciliary muscle. Compound 1 was cumulatively added beginning the point of time when the muscle contractility had become steady.

(4) Results

As shown in Fig. 1, with the $10^{-4}$ M carbachol-induced contraction of the rabbit ciliary muscle being taken as 100, Compound 1 induced the muscle to start relaxing at $10^{-6}$ M and caused a relaxation of about 42% at $10^{-4}$ M.

Test Example 2 (the ciliary muscle relaxing effect of the chymase inhibitor in dogs)

(1) Experimental animals

Male beagle dogs weighing about 7 kg were used.

(2) Test drugs

Ciliary muscle relaxant: chymase inhibitor
Ciliary muscle constricting agent: carbachol ($10^{-4}$ M)
As the chymase inhibitor, 2-[1,6-dihydro-5-hydroxysuccinylamino-2-(3-methylphenyl)-6-oxo-1-pyrimidinyl]-N-[1-benzyl-3,3-difluoro-2-oxo-3-[N-(carboxymethyl)carbamoyl] propyl]acetamide (Compound 2) was used.
Compound 2 and carbachol were dissolved in Krebs-Henseleit solution.

(3) Test protocol

The eyeball was enucleated and a 2~3 mm-wide x 4~5 mm-long ciliary muscle specimen in radial direction was prepared. The specimen was suspended in a Magnus tube (10 mL) filled with Krebs-Henseleit solution aerated with 95% $O_2$-5% $CO_2$, and equilibrated for 30 minutes under a static tension of 50 mg. The change in tension was isometrically recorded on a recorder (U-228, manufactured by Nippon Denko Kagaku) through a transducer (TB-612T, manufactured by Nippon Kohden) and an amplifier (SEN-6102, manufactured by Nippon Kohden) to evaluate the muscle relaxant effect of Compound 2 on carbachol ($10^{-4}$ M)-induced persistent contraction of the canine ciliary muscle. Compound 2 was cumulatively added beginning the point of time when the muscle contractility had become steady.

(4) Results

As shown in Fig. 2, with the carbachol ($10^{-4}$ M)-induced persistent contraction of the canine ciliary muscle being taken as 100, Compound 2 ($10^{-5}$ M) inhibited the contraction significantly, reducing the tension of the ciliary muscle.

INDUSTRIAL APPLICABILITY

The ciliary muscle relaxant according to the present invention is of value as a prophylactic or therapeutic drug for various types of myopia arising from hypertonia of the ciliary muscle, such as pseudomyopia, low myopia, and high

myopia.

## Claims

1. A ciliary muscle relaxant comprising a chymase inhibitor as an active ingredient.

2. The ciliary muscle relaxant according to Claim 1 wherein the chymase inhibitor is an imidazolidine derivative represented by the formula (IV)

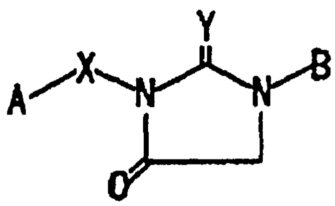

(IV)

(A and B each represents an aromatic hydrocarbon group, which may be substituted by 1-3 groups selected from among halogen, $C_{1-4}$ alkyl, $C_{1-4}$ alkoxy, $C_{1-4}$ alkylenedioxy, phenoxy, nitro, cyano, phenyl, $C_{2-5}$ alkanoylamino, carboxy optionally esterified with a $C_{1-4}$ alkyl or alkenyl group, carboxyalkyl optionally esterified with a $C_{1-4}$ alkyl or alkenyl group; carboxyalkyloxy optionally esterified with a $C_{1-4}$ alkyl or alkenyl group, N-alkylpiperazinylcarbonyl, N-alkylpiperazinylcarbonylalkyl, N-alkylpiperazinylcarbonylalkyloxy, and morpholinocarbonyl; X represents sulfonyl or carbonyl; Y represents oxygen or sulfur).

3. The ciliary muscle relaxant according to Claim 2 wherein the chymase inhibitor is a compound of the formula (IV) wherein A is an aromatic hydrocarbon group which may optionally be substituted with halogen; B is an aromatic hydrocarbon group; X is sulfonyl; and Y is oxygen.

4. The ciliary muscle relaxant according to Claim 1 wherein the chymase inhibitor is 3-(4-chlorobenzenesulfonyl)-1-phenylimidazolidine-2,4-dione.

5. The ciliary muscle relaxant according to Claim 1 wherein the chymase inhibitor is a heterocyclic amide compound represented by the formula (XI)

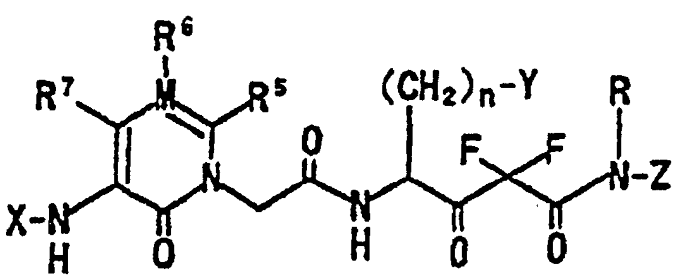

(XI)

[wherein X represents $-COOR^1$, $-(CH_2)_mCOOR^1$, or $-CO(CH_2)_mCOOR^1$ ($R^1$ represents hydrogen, alkyl, cycloalkyl, cycloalkylalkyl, aryl, arylalkyl, heteroaryl, heteroarylalkyl, heterocycle, or heterocyclealkyl; m represents 1, 2, or 3); Z represents $-(CH_2)_rCOOR^2$ or a group of the formula (i):

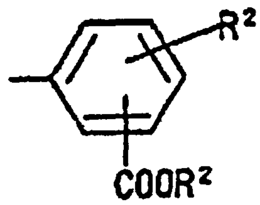

(i)

(wherein $R^2$ represents hydrogen, alkyl, cycloalkyl, cycloalkylalkyl, aryl, arylalkyl, heteroarylalkyl, heterocycle, or heterocyclealkyl; $R^3$ represents hydrogen, alkyl, alkoxy, or halogen; r represents 1, 2, or 3); R represents hydrogen or alkyl; $R^5$, $R^6$ and $R^7$ may be the same or different and each independently represents hydrogen or alkyl, or one of $R^5$, $R^6$ and $R^7$ represents aryl, arylalkyl, arylalkenyl, heteroaryl, heteroarylalkyl or heteroarylalkenyl, while the remaining others each represents hydrogen; M represents carbon or nitrogen; provided, however, that in the case where M is nitrogen, $R^6$ does not exist; Y represents cycloalkyl, aryl, or heteroaryl; n represents 0 or 1; among the above groups, said aryl, arylalkyl, heteroaryl and heteroarylalkyl may each be substituted] or a pharmacologically acceptable salt thereof.

**6.** The ciliary muscle relaxant according to Claim 5 wherein the chymase inhibitor is a compound of the formula (XI) wherein X is $-CO(CH_2)_mCOOH$ (m is 1, 2, or 3), Y is aryl, Z is $-CO(CH_2)_rCOOH$ (r is 1, 2, or 3), M is nitrogen, $R^5$ is aryl which may be substituted with alkyl, and $R^7$ is hydrogen.

**7.** The ciliary muscle relaxant according to Claim 1 wherein the chymase inhibitor is 2-[1,6-dihydro-5-hydroxysuccinylamino-2-(3-methylphenyl)-6-oxo-1-pyrimidinyl]-N-[1-benzyl-3,3-difluoro-2-oxo-3-[N-(carboxymethyl)carbamoyl] propyl]acetamide.

**8.** The ciliary muscle relaxant according to any of Claims 1-7 which is for the prophylaxis or therapy of myopia.

**9.** The ciliary muscle relaxant according to any of Claims 1-7 which is in the form of a topical ophthalmic preparation.

**10.** A pharmaceutical composition for reducing the tension of the ciliary muscle which comprises a chymase inhibitor and a pharmaceutically acceptable carrier or excipient.

**11.** The pharmaceutical composition according to Claim 10 wherein the chymase inhibitor is an imidazolidine derivative of the formula (IV)

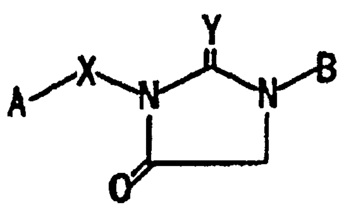

(IV)

(A and B each represents an aromatic hydrocarbon group, which may be substituted by 1-3 groups selected from among halogen, $C_{1-4}$ alkyl, $C_{1-4}$ alkoxy, $C_{1-4}$ alkylenedioxy, phenoxy, nitro, cyano, phenyl, $C_{2-5}$ alkanoylamino, carboxy optionally esterified with a $C_{1-4}$ alkyl or alkenyl group, carboxyalkyl optionally esterified with a $C_{1-4}$ alkyl or alkenyl group; carboxyalkyloxy optionally esterified with a $C_{1-4}$ alkyl or alkenyl group, N-alkylpiperazinylcarbonyl, N-alkylpiperazinylcarbonylalkyl, N-alkylpiperazinylcarbonylalkyloxy, and morpholinocarbonyl; X represents sulfonyl or carbonyl; Y represents oxygen or sulfur).

**12.** The pharmaceutical composition according to Claim 11 wherein the chymase inhibitor is a compound of the formula (IV) wherein A is an aromatic hydrocarbon group which may optionally be substituted with halogen; B is an aromatic hydrocarbon group; X is sulfonyl; and Y is oxygen.

**13.** The pharmaceutical composition according to Claim 10 wherein the chymase inhibitor is 3-(4-chlorobenzenesulfonyl)-1-phenylimidazolidine-2,4-dione.

**14.** The pharmaceutical composition according to Claim 10 wherein the chymase inhibitor is a heterocyclic amide compound represented by the formula (XI)

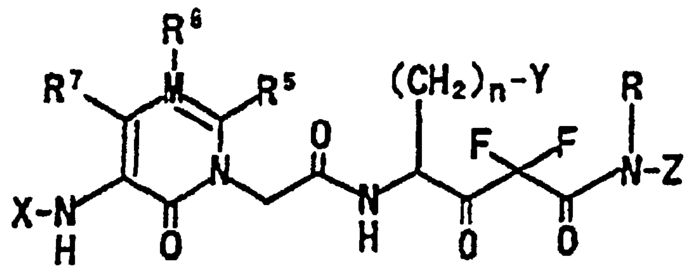

(XI)

[wherein X represents $-COOR^1$, $-(CH_2)_mCOOR^1$, or $-CO(CH_2)_mCOOR^1$ ($R^1$ represents hydrogen, alkyl, cycloalkyl, cycloalkylalkyl, aryl, arylalkyl, heteroaryl, heteroarylalkyl, heterocycle, or heterocyclealkyl; m represents 1, 2, or 3); Z represents $-(CH_2)_rCOOR^2$ or a group of the formula (i):

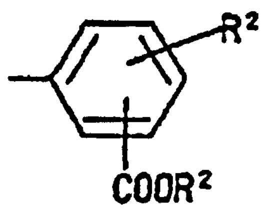

(i)

(wherein $R^2$ represents hydrogen, alkyl, cycloalkyl, cycloalkylalkyl, aryl, arylalkyl, heteroarylalkyl, heterocycle, or heterocyclealkyl; $R^3$ represents hydrogen, alkyl, alkoxy, or halogen; r represents 1, 2, or 3); R represents hydrogen or alkyl; $R^5$, $R^6$ and $R^7$ may be the same or different and each independently represents hydrogen or alkyl, or one of $R^5$, $R^6$ and $R^7$ represents aryl, arylalkyl, arylalkenyl, heteroaryl, heteroarylalkyl or heteroarylalkenyl, while the remaining others each represents hydrogen; M represents carbon or nitrogen; provided, however, that in the case where M is nitrogen, $R^6$ does not exist; Y represents cycloalkyl, aryl, or heteroaryl; n represents 0 or 1; among the above groups, said aryl, arylalkyl, heteroaryl and heteroarylalkyl may each be substituted] or a pharmacologically acceptable salt thereof.

**15.** The pharmaceutical composition according to Claim 14 wherein the chymase inhibitor is a compound of the formula (XI) wherein X is $-CO(CH_2)_mCOOH$ (m is 1, 2, or 3), Y is aryl, Z is $-CO(CH_2)_rCOOH$ (r is 1, 2, or 3), M is nitrogen, $R^5$ is aryl which may be substituted with alkyl, and $R^7$ is hydrogen.

**16.** The pharmaceutical composition according to Claim 10 wherein the chymase inhibitor is 2-[1,6-dihydro-5-hydroxysuccinylamino-2-(3-methylphenyl)-6-oxo-1-pyrimidinyl]-N-[1-benzyl-3,3-difluoro-2-oxo-3-[N-(carboxymethyl) carbamoyl] propyl]acetamide.

**17.** The pharmaceutical composition according to any of Claims 10-16 for the prophylaxis or therapy of myopia.

**18.** The pharmaceutical composition according to any of Claims 10-17 which is in the form of a topical ophthalmic preparation.

**19.** Use of a chymase inhibitor for the production of a pharmaceutical composition for reducing the tension of the ciliary muscle.

**20.** The use according to Claim 19 wherein the chymase inhibitor is an imidazolidine derivative represented by the formula (IV)

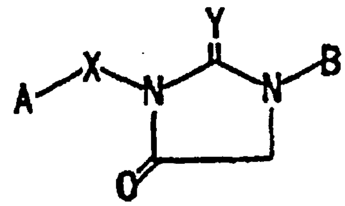

(IV)

(A and B each represents an aromatic hydrocarbon group, which may be substituted by 1-3 groups selected from among halogen, $C_{1-4}$ alkyl, $C_{1-4}$ alkoxy, $C_{1-4}$ alkylenedioxy, phenoxy, nitro, cyano, phenyl, $C_{2-5}$ alkanoylamino, carboxy optionally esterified with a $C_{1-4}$ alkyl or alkenyl group, carboxyalkyl optionally esterified with a $C_{1-4}$ alkyl or alkenyl group; carboxyalkyloxy optionally esterified with a $C_{1-4}$ alkyl or alkenyl group, N-alkylpiperazinylcarbonyl, N-alkylpiperazinylcarbonylalkyl, N-alkylpiperazinylcarbonylalkyloxy, and morpholinocarbonyl; X represents sulfonyl or carbonyl; Y represents oxygen or sulfur).

**21.** The use according to Claim 20 wherein the chymase inhibitor is a compound of the formula (IV) wherein A is an aromatic hydrocarbon group which may optionally be substituted with halogen; B is an aromatic hydrocarbon group; X is sulfonyl; and Y is oxygen.

**22.** The use according to Claim 19 wherein the chymase inhibitor is 3-(4-chlorobenzenesulfonyl)-1-phenylimidazolidine-2,4-dione.

**23.** The use according to Claim 19 wherein the chymase inhibitor is a heterocyclic amide compound represented by the formula (XI)

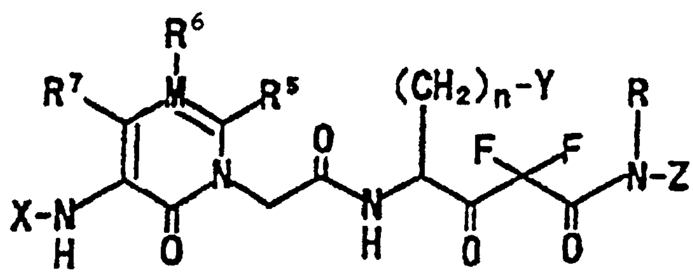

(XI)

[wherein X represents $-COOR^1$, $-(CH_2)_mCOOR^1$, or $-CO(CH_2)_mCOOR^1$ ($R^1$ represents hydrogen, alkyl, cycloalkyl, cycloalkylalkyl, aryl, arylalkyl, heteroaryl, heteroarylalkyl, heterocycle, or heterocyclealkyl; m represents 1, 2, or 3); Z represents $-(CH_2)_rCOOR^2$ or a group of the formula (i):

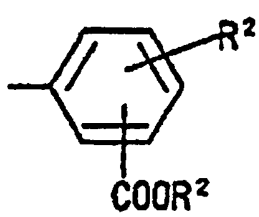

(i)

(wherein $R^2$ represents hydrogen, alkyl, cycloalkyl, cycloalkylalkyl, aryl, arylalkyl, heteroarylalkyl, heterocycle, or heterocyclealkyl; $R^3$ represents hydrogen, alkyl, alkoxy, or halogen; r represents 1, 2, or 3); R represents hydrogen or alkyl; $R^5$, $R^6$ and $R^7$ may be the same or different and each independently represents hydrogen or alkyl, or one of $R^5$, $R^6$ and $R^7$ represents aryl, arylalkyl, arylalkenyl, heteroaryl, heteroarylalkyl or heteroarylalkenyl, while the remaining others each represents hydrogen; M represents carbon or nitrogen; provided, however, that in the case where M is nitrogen, $R^6$ does not exist; Y represents cycloalkyl, aryl, or heteroaryl; n represents 0 or 1; among the

above groups, said aryl, arylalkyl, heteroaryl and heteroarylalkyl may each be substituted] or a pharmacologically acceptable salt thereof.

**24.** The use according to Claim 23 wherein the chymase inhibitor is a compound of the formula (XI) wherein X is -CO $(CH_2)_mCOOH$ (m is 1, 2, or 3), Y is aryl, Z is $-CO(CH_2)_rCOOH$ (r is 1, 2, or 3), M is nitrogen, $R^5$ is aryl which may be substituted with alkyl, and $R^7$ is hydrogen.

**25.** The use according to Claim 19 wherein the chymase inhibitor is 2-[1,6-dihydro-5-hydroxysuccinylamino-2-(3-methylphenyl)-6-oxo-1-pyrimidinyl]-N-[1-benzyl-3,3-difluoro-2-oxo-3-[N-(carboxymethyl)carbamoyl]propyl]acetamide.

**26.** The use of a chymase inhibitor according to any of Claims 19-25 wherein the pharmaceutical product is for reducing the tension of the ciliary muscle is indicated for the prophylaxis or therapy of myopia.

**27.** The use of a chymase inhibitor according to any of Claims 19-26 wherein the pharmaceutical product is a topical ophthalmic preparation.

**28.** A method of reducing the tension of the ciliary muscle which comprises administering an effective amount of a chymase inhibitor to a patient requiring it.

**29.** The method of reducing the tension of the ciliary muscle according to Claim 28 wherein the chymase inhibitor is an imidazolidine derivative represented by the formula (IV)

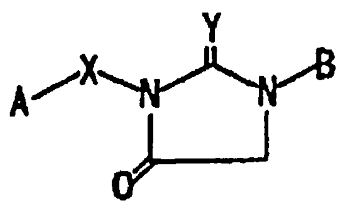

(IV)

(A and B each represents an aromatic hydrocarbon group, which may be substituted by 1-3 groups selected from among halogen, $C_{1-4}$ alkyl, $C_{1-4}$ alkoxy, $C_{1-4}$ alkylenedioxy, phenoxy, nitro, cyano, phenyl, $C_{2-5}$ alkanoylamino, carboxy optionally esterified with a $C_{1-4}$ alkyl or alkenyl group, carboxyalkyl optionally esterified with a $C_{1-4}$ alkyl or alkenyl group; carboxyalkyloxy optionally esterified with a $C_{1-4}$ alkyl or alkenyl group, N-alkylpiperazinylcarbonyl, N-alkylpiperazinylcarbonylalkyl, N-alkylpiperazinylcarbonylalkyloxy, and morpholinocarbonyl; X represents sulfonyl or carbonyl; Y represents oxygen or sulfur).

**30.** The method of reducing the tension of the ciliary muscle according to Claim 29 wherein the chymase inhibitor is a compound of the formula (IV) wherein A is an aromatic hydrocarbon group which may optionally be substituted with halogen; B is an aromatic hydrocarbon group; X is sulfonyl; and Y is oxygen.

**31.** The method of reducing the tension of the ciliary muscle according to Claim 28 wherein the chymase inhibitor is 3-(4-chlorobenzenesulfonyl)-1-phenylimidazolidine-2,4-dione.

**32.** The method of reducing the tension of the ciliary muscle according to Claim 28 wherein the chymase inhibitor is a heterocyclic amide compound represented by the formula (XI)

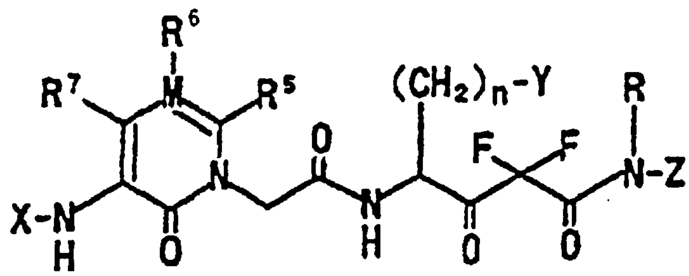

(XI)

[wherein X represents $-COOR^1$, $-(CH_2)_mCOOR^1$, or $-CO(CH_2)_mCOOR^1$ ($R^1$ represents hydrogen, alkyl, cycloalkyl, cycloalkylalkyl, aryl, arylalkyl, heteroaryl, heteroarylalkyl, heterocycle, or heterocyclealkyl; m represents 1, 2, or 3); Z represents $-(CH_2)_rCOOR^2$ or a group of the formula (i):

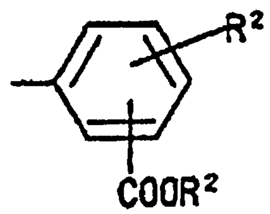

(i)

(wherein $R^2$ represents hydrogen, alkyl, cycloalkyl, cycloalkylalkyl, aryl, arylalkyl, heteroarylalkyl, heterocycle, or heterocyclealkyl; $R^3$ represents hydrogen, alkyl, alkoxy, or halogen; r represents 1, 2, or 3); R represents hydrogen or alkyl; $R^5$, $R^6$ and $R^7$ may be the same or different and each independently represents hydrogen or alkyl, or one of $R^5$, $R^6$ and $R^7$ represents aryl, arylalkyl, arylalkenyl, heteroaryl, heteroarylalkyl or heteroarylalkenyl, while the remaining others each represents hydrogen; M represents carbon or nitrogen; provided, however, that in the case where M is nitrogen, $R^6$ does not exist; Y represents cycloalkyl, aryl, or heteroaryl; n represents 0 or 1; among the above groups, said aryl, arylalkyl, heteroaryl and heteroarylalkyl may each be substituted] or a pharmacologically acceptable salt thereof.

**33.** The method of reducing the tension of the ciliary muscle according to Claim 32 wherein the chymase inhibitor is a compound of the formula (XI) wherein X is $-CO(CH_2)_mCOOH$ (m is 1, 2, or 3), Y is aryl, Z is $-CO(CH_2)_rCOOH$ (r is 1, 2, or 3), M is nitrogen, $R^5$ is aryl which may be substituted with alkyl, and $R^7$ is hydrogen.

**34.** The method of reducing the tension of the ciliary muscle according to Claim 28 wherein the chymase inhibitor is 2-[1,6-dihydro-5-hydroxysuccinylamino-2-(3-methylphenyl)-6-oxo-1-pyrimidinyl]-N-[1-benzyl-3,3-difluoro-2-oxo-3-[N-(carboxymethyl)carbamoyl]propyl]acetamide.

**35.** The method of reducing the tension of the ciliary muscle according to any of Claims 28-34 for the prophylaxis or therapy of myopia.

**36.** The method of reducing the tension of the ciliary muscle according to any of Claims 28-35 which comprises administering the chymase inhibitor to the patient's eye requiring it.

Fig 1

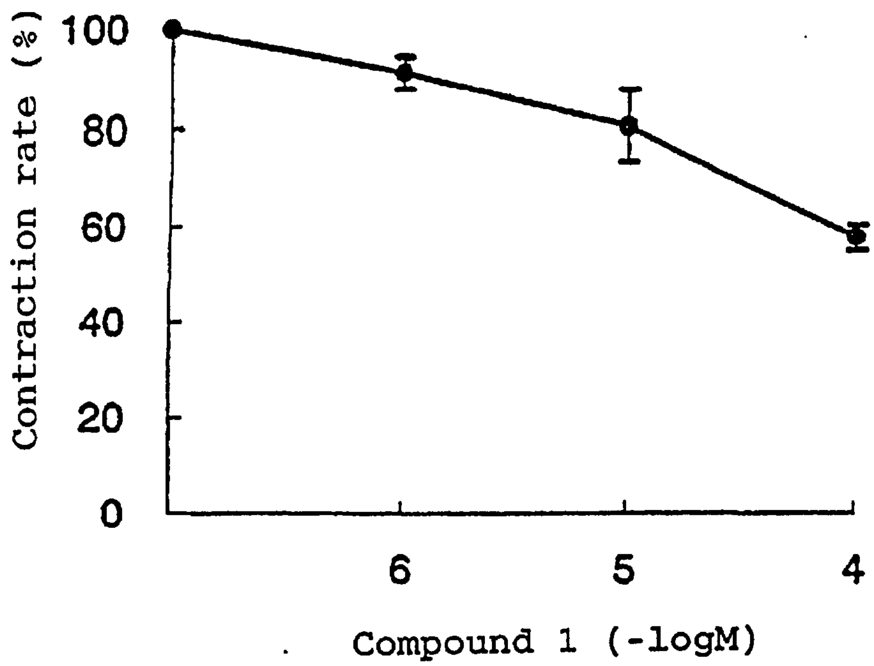
Contraction rate (%)
100
80
60
40
20
0
6
5
4
Compound 1 (-logM)

Fig 2

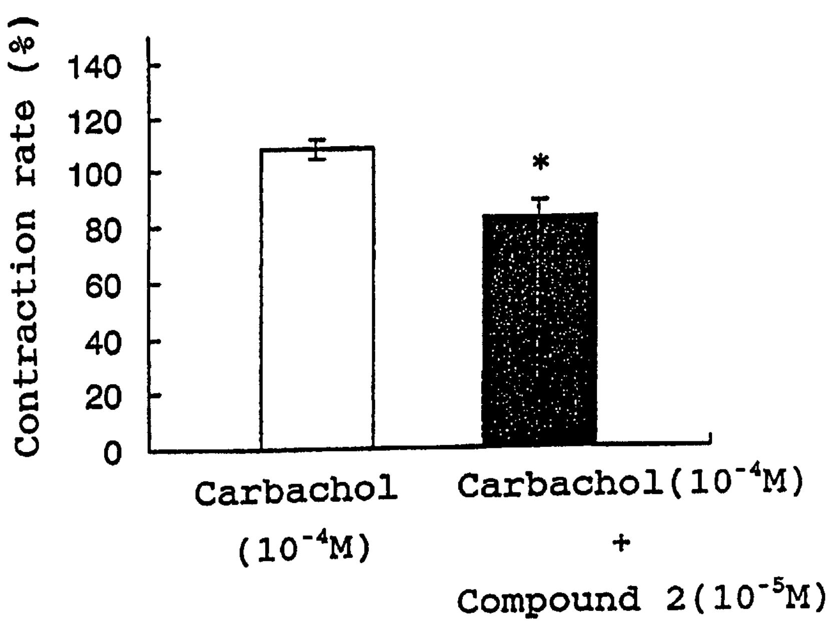
Contraction rate (%)
140
120
100
80
60
40
20
0
*
Carbachol
($10^{-4}$M)
Carbachol($10^{-4}$M)
+
Compound 2($10^{-5}$M)

**INTERNATIONAL SEARCH REPORT**

International application No.
PCT/JP00/07390

A. CLASSIFICATION OF SUBJECT MATTER
Int.Cl$^7$ A61K45/00, 31/4166, 31/513, A61P27/10, 43/00

According to International Patent Classification (IPC) or to both national classification and IPC

B. FIELDS SEARCHED

Minimum documentation searched (classification system followed by classification symbols)
Int.Cl$^7$ A61K45/00, 31/4166, 31/513, A61P27/10, 43/00

Documentation searched other than minimum documentation to the extent that such documents are included in the fields searched

Electronic data base consulted during the international search (name of data base and, where practicable, search terms used)
CAPLUS(STN),MEDLINE(STN),EMBASE(STN)

C. DOCUMENTS CONSIDERED TO BE RELEVANT

| Category* | Citation of document, with indication, where appropriate, of the relevant passages | Relevant to claim No. |
|---|---|---|
| A | JP, 9-59173, A (SANTEN PHARMACEUTICAL CO., LTD.), 04 March, 1997 (04.03.97) (Family: none) & Database CAPLUS on STN,AMERICAN CHEMICAL SOCIETY (ACS),(Columbus,OH,USA),DN.126:282796 | 1-27 |
| A | WO, 96/33974, A1 (THE GREEN CROSS CORPORATION), 31 October, 1996 (31.10.96) & EP, 826671, A1 & US, 5948785, A | 1-27 |
| A | WO, 96/04248, A1 (SUNTORY LTD.), 15 February, 1996 (15.02.96) & EP, 721944, A1 & US, 5691335, A | 1-27 |
| A | JP, 10-53579, A (Fujisawa Pharmaceutical Co., Ltd.), 24 February, 1998 (24.02.98) (Family: none) & Database CAPLUS on STN, AMERICAN CHEMICAL SOCIETY (ACS),(Columbus,OH,USA),DN.128:192666 | 1-27 |
| A | WO, 95/21609, A1 (CIBA-GEIGY A.-G.), 17 August, 1995 (17.08.95) & EP, 743852, A1 & US, 5889020, A | 1-27 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 December, 2000 (19.12.00) | 26 December, 2000 (26.12.00) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/07390

**Box I Observations where certain claims were found unsearchable (Continuation of item 1 of first sheet)**

This international search report has not been established in respect of certain claims under Article 17(2)(a) for the following reasons:

1. ☒ Claims Nos.: 28-36
because they relate to subject matter not required to be searched by this Authority, namely:

Claims 28 to 36 pertain to methods for treatment of the human body by therapy and thus relate to a subject matter which this International Searching Authority is not required, under the provisions of Article 17(2)(a)(i) of the PCT and Rule 39(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)